# EUROPEAN PATENT SPECIFICATION

(11) **EP 4 314 558 B1**
(45) Date of publication and mention of the grant of the patent: **28.05.2025**
(21) Application number: 22711551.6
(22) Date of filing: 09.03.2022
(51) Int. Cl.: F04B 43/12, A61M 60/109, A61M 60/279, A61M 60/441

(54) **PERISTALTIC PUMP EQUIPPED WITH A RELEASE SYSTEM TO DISENGAGE THE FLUID TUBE**
PERISTALTISCHE PUMPE MIT AUSLÖSUNGSSYSTEM ZUM ENTFERNEN DES SCHLAUCHES
POMPE PÉRISTALTIQUE AVEC UN SYSTÈME DE LIBÉRATION POUR DÉSENGAGER LE TUYAU DE FLUIDE

(30) Priority: 25.03.2021 IT 202100007358
(43) Date of publication of application: 07.02.2024
(73) Proprietor: BAXTER HEALTHCARE SA, 8152 Glattpark (Opfikon) (CH); Baxter International Inc, Deerfield, IL 60015 (US)
(72) Inventor: PARALUPPI, Marco, 41036 Medolla (MO) (IT)
(74) Representative: PGA S.p.A., Milano, Succursale di Lugano
(86) International application number: PCT/EP2022/056101
(87) International publication number: WO 2022/200050

(56) References cited:
- WO-A1-2014/053858
- WO-A1-91/16542
- DE-A1- 1 807 979
- US-A- 3 787 148
- US-A1- 2019 047 295

## Description

### FIELD OF THE INVENTION

The present invention relates to a peristaltic pump having a releasable system to disengage a fluid tube.

The invention also relates to a method to switch the peristaltic pump from an operative configuration to a released configuration and vice versa.

Furthermore, the invention also relates to a method to balance fluid pressures in the fluid line, upstream and downstream the peristaltic pump of the present invention.

### BACKGROUND

Peristaltic pumps are commonly used to promote fluid flow in a fluid line: in particular peristaltic pumps are used in medical field because of safety reasons as these pumps allow to prevent the fluid from contacting external parts other than the line, thereby improving sterilization.

In common peristaltic pumps, rollers or shoes compress the flexible line as they rotate, creating a negative pressure behind that recalls the fluid, and a positive pressure forward causing the fluid to advance. Common peristaltic pumps comprise a rotor carrying the rollers and a stator carrying a pump race partially surrounding the rotor and defining a gap in between: the flexible line is operatively arranged within this gap so that the line is compressed, in an operative condition, between a roller and the external pump race. Rotation of the rotor determines advancing of the roller with respect to the race over the line, causing the fluid in the line to advance.

During operations, it may be requested to periodically remove and reinstall the fluid line between the race and the rollers. Removal and reinstallation procedures imply to squeeze and forcibly manipulate the fluid line, as the latter is compressed or has to be compressed between the rollers and the surrounding race, thereby stressing repetitively the fluid line: manipulation may lead to premature damage of the fluid line causing the need of replacing it and negatively affecting the safety level of the pump system. Notably, a damage occurring to a blood line, such as a crack on the line, during an extracorporeal blood treatment, may lead to extremely dangerous consequences for the patient, such as massive blood loss or allowing bacteria to enter the blood stream.

Furthermore, as the fluid line is compressed between the rollers and the track, installation and removal procedures may result to be challenging, thereby requiring the intervention of a professional person, i.e. a nurse or a doctor, and extending the time required to accomplish the task.

SCOPE OF THE INVENTION DE1807979 discloses a peristaltic pump in accordance with the preamble of claim 1. The scope of this invention is therefore to at least partially solve one or more of the drawbacks and/or limitations of the prior art solutions.

A first scope is to provide a peristaltic pump able to substantially reduce or completely remove the risk of damaging the fluid line during periodical installation and removal procedures.

A further scope is to provide a peristaltic pump able to ease periodical installation and removal procedures of a fluid line without requiring massive manipulation.

A further scope is to provide a peristaltic pump combining manufacturing costs low and high reliability with the preceding features.

These scopes and more, which will appear more from the following description, are substantially achieved by a peristaltic pump in accordance with one or more of the following claims

### DRAWINGS

Some embodiments and some aspects of the invention will be described below with reference to the attached drawings, provided for illustrative purposes only, wherein:
- Figure 1 is a perspective view of a peristaltic pump according to the present invention with the tube line installed;
- Figures 2 and 3 are front views of a peristaltic pump according to the present invention in an operative and released configuration respectively;
- Figure 4 is a perspective view of the rotor of a peristaltic pump according to the present invention in an operative position;
- Figure 5 is a detailed view of the peristaltic pump according to the present invention in a released configuration;
- Figure 6 is a front view of a peristaltic pump according to the present invention in a released configuration with part of the rotor unmounted;
- Figure 7 is a perspective view of a peristaltic pump according to the present invention with the rotor unmounted;
- Figure 8 is an exploded view of a peristaltic pump according to the present invention;
- Figure 9 is a flow chart representing method steps related to the peristaltic pump of the present invention.

### DEFINITIONS

In this detailed description, corresponding parts illustrated in the various figures are indicated with the same numerical references. The figures may illustrate the invention by means of non-scale representations; therefore, parts and components illustrated in the figures relating to the object of the invention may relate exclusively to schematic representations.

### Upstream and/ downstream

The terms upstream and downstream refer to a direction or trajectory of advancement of a fluid configured to flow within the fluid line during standard usage of the peristaltic pump. In particular the fluid is configured to flow from an upstream tract to a downstream tract of the fluid line: the pump is interposed between the downstream and the upstream tract of the fluid line. The upstream tract is commonly known as the supply line, while the downstream tract is commonly known as the delivery line.

### DETAILED DESCRIPTION

### Peristaltic pump

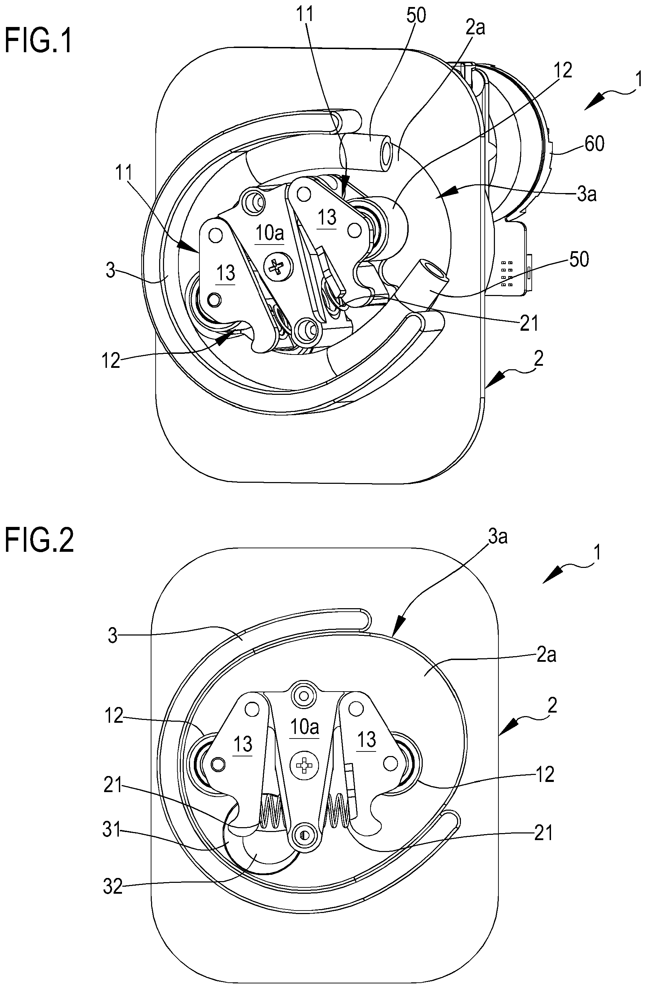

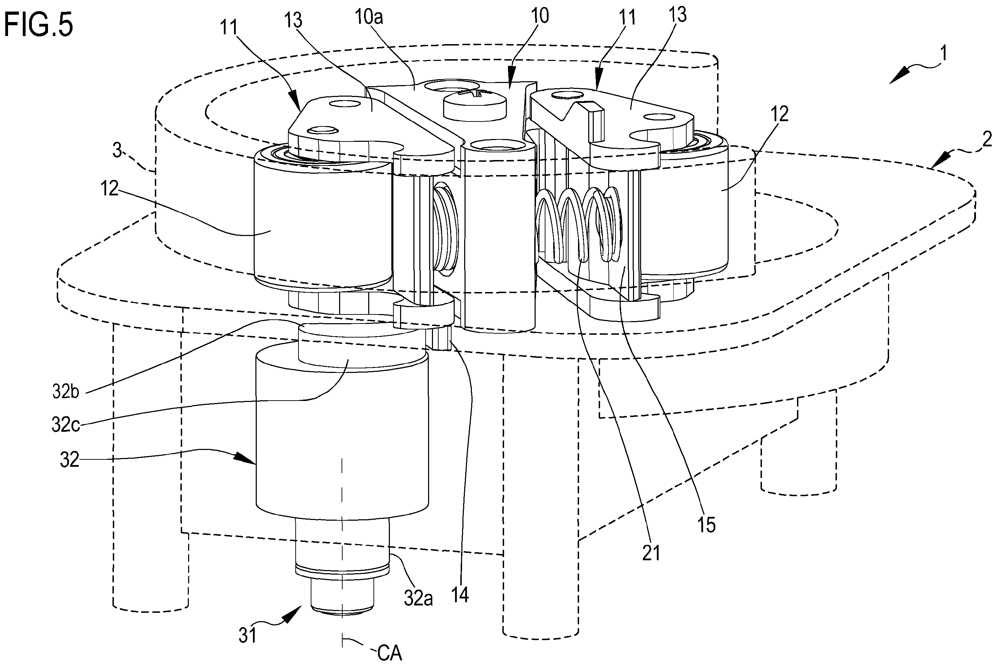

Reference number 1 is directed to a peristaltic pump as shown in figures 1-8. The working principle of a peristaltic pump is well known to a skilled person: thus, the features described here after and associated to the main components of a common peristaltic pump have to be read as exemplary, as the skilled person is able to provide further modification non relating to the key concept of the present invention, which necessarily involves the presence of a releasing system 30 configured to ease installation and removal procedures of a fluid line.

The peristaltic pump of the present invention may be used for several purposes in a broad range of technical fields. In particular, the peristaltic pump of the present invention is directed to the medical field, in particular to dialysis field, wherein the fluid line may transport blood, dialysis fluid, replacement fluid, infusion fluid or the like. Normally, peristaltic pump in medical field are configured to provide a fluid rate for blood within a range between 50ml/min and 500ml/min, more in particular between 150ml/min and 400ml/min. Note that usually blood flow rate is paired with a dialysate flow of about double the blood flow rate (e.g. 100ml/min to 1000ml/min - 300ml/min to 800ml/min).

Peristaltic pump 1 is configured to receive a fluid line 50 having a diameter comprised between 3mm and 8mm: the fluid line may be made by plastic or silicone material. The main components of the peristaltic pump 1 are the stator 2, which defines the static body of the pump, and the rotor 10 which is rotatable about a rotor axis RA: the stator 2 carries the rotor 10. An exemplary peristaltic pump 1 with a tube line installed is shown in figure 1.

The stator 2 comprises a base wall 2a and a pump race 3 emerging in height from the base wall 2a, defining an internal volume 5: in particular the pump race 3 emerges substantially normally with respect to the base wall 2a. The pump race 3 may have a height comprised between 3mm and 20mm. The base wall may have a substantially flat shape or slightly conic, while the pump race 3 may have a semicircular shape, i.e. "C" or "U" shape, concentric to the rotor axis RA defining an internal wall configured to receive in abutment the fluid line: the fluid line, when arranged in the stator 2, defines a loop as in figure 1. The internal wall of the pump race 3 defines an operative arc wherein the rotor 10 is configured to operatively cooperate with the fluid line to promote fluid flow within the fluid line. The internal volume 5 of the stator 2 is laterally confined by this internal wall of the pump race 3 and confined on the bottom by the base wall 2a of the stator 2.

The pump race 3 also has a lateral aperture 3a, as shown in figures 1 and 2, configured to allow an inlet tract of the fluid line 50 to enter in the inner volume of the pump, and an exit tract of the fluid line to exit the pump. The lateral aperture defines a non-operative arc wherein the fluid line does not cooperate with the rotor: The non-operative arc may have an angular dimension comprised between 20° and 200°, more in particular between 45° and 180°, more in particular between 60° and 135°.

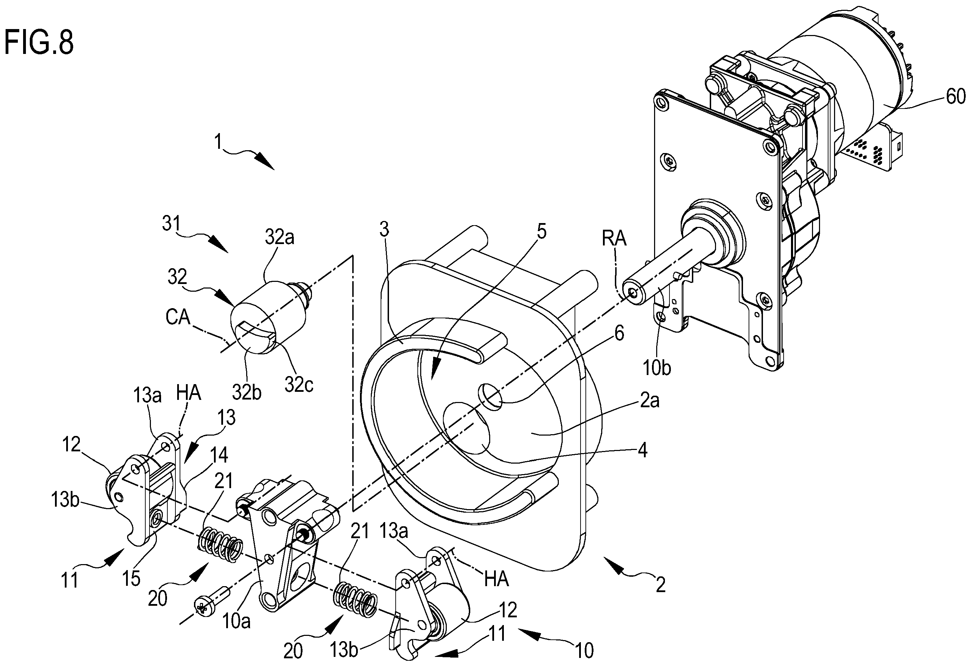

The stator 2 also has a through aperture 6 defining an aperture axis AA coincident with the rotor axis RA: the through aperture 6 is configured to bear a rotor axle 10b of the rotor 10, as shown in the exploded view of figure 8.

The stator 2 may be made by plastic or metallic material.

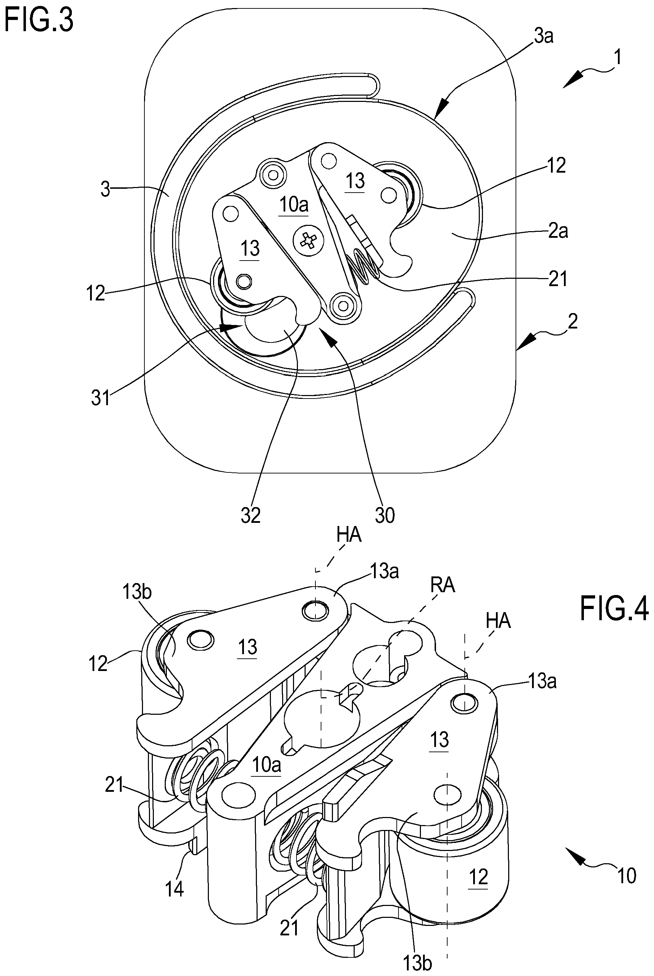

The rotor 10 of the present peristaltic pump 1 is surrounded at least partially by the pump race 3 and configured to rotate about the rotor axis RA. The rotor 10 is arranged at least partially in the internal volume 5 of the stator and may include a rotor axle 10b, carried by the stator and arranged in the through aperture 6 of the stator, and a rotor plate 10a coupled or coupleable to the rotor axle. The rotor 10 includes at least one contrasting element 11, carried by the rotor plate 10a, configured to abut against the fluid line 50 and also rotatable about the rotor axis RA. The at least one contrasting element 11 is movable towards and away with respect to the rotor axis RA and the pump race 3 between a released position shown in figures 3, 5 and 6, wherein the at least one contrasting element 11 is away from the pump race 3, and an operative position shown in figures 1, 2, 3 wherein the at least one contrasting element 11 is approached to the pump race 3 and configured to abut against the fluid line 50. Notably, in the released position, the contrasting element 11 is at a distance from the pump race 3 higher than a corresponding distance in the operative position. In particular the contrasting element 11, when arranged in the released position, defines a distance with respect to the inner wall of the pump race 3 comprised between 0.7 and two times an external diameter of the fluid line, more in particular between one and 1.5 times an external diameter of the fluid line. More in detail, this distance is comprised between 3mm and 8mm. In other terms, a contrasting element 11, when arranged in the released position, is configured to reduce or completely remove a compression force on the fluid line 50 when the latter is installed on the pump 1.

On the other hand, the contrasting element 11, when arranged in the operative position, defines a distance with respect to the inner wall of the pump race 3 comprised between 2mm and 3mm, in order to compress the fluid line 50 against the pump race.

In an embodiment, the peristaltic pump 1 comprises two or more contrasting elements 11: in particular the contrasting elements 11 may be in a number comprised between two and ten, more in particular between two and six according to design requirements.

Each contrasting element 11 may include at least one roller or shoe 12 configured to contact and squeeze the fluid line 50 against the pump race 3, as shown in figure 1. Figures 1 show an embodiment of the pump using rollers 12 in order to reduce tangential stresses on the fluid line.

Each contrasting element 11 comprises a lever arm 13 extending in length between a hinged end 13a and a free end 13b, wherein the hinged end 13a is hinged to the rotor plate 10a, and the free end 13b carries the roller or shoe 12. The lever arm 13 is rotatable about the hinged end 13a about a hinge axis HA and with respect to the rotor plate 10a: a rotation of the lever arm 13 about the hinge axis HA determines the passage of the respective contrasting element 11 between the operative position and the released position and vice versa.

In an embodiment wherein the pump comprises two contrasting elements 11, the two contrasting elements 11 are arranged diametrically opposite each other, namely defining a 180° angle in between. In a further embodiment wherein the pump comprises three or more contrasting elements 11, the contrasting elements 11 may be angularly spaced each other in a substantially evenly manner, in particular wherein the three or more contrasting elements are equally spaced each other.

The lever arm 13 has an extended body and may have an abutment protrusion 14 emerging from the extended body: the abutment protrusion 14 is interposed between the extended body and the base wall 2a of the stator 2. The abutment protrusion 14 may emerge from the extended body of the lever arm 13 towards the base wall 2a of the stator 2, so that the abutment protrusion 14 is the closest portion of the lever arm 13 to the base wall 2a of the stator 2. In other terms, the abutment protrusion 14 reduces a distance between the lever arm 13 and the base wall 2a of the stator 2: the distance between the abutment protrusion 14 and the base wall 2a may be comprised between 1,5mm and 10 or more mm.

In an embodiment, only one contrasting element 11 of the two or more contrasting elements comprises the abutment protrusion 14 interposed between the extended body and the base wall 2a of the stator 2 and engageable by a releasing member 31 described here after. In a further embodiment, two contrasting elements 11 of the two or more contrasting elements comprise respective abutment protrusions 14 interposed between the extended body and the base wall 2a of the stator 2 and engageable by the releasing member 31. In a still further embodiment, all the contrasting elements 11 of the two or three or more contrasting elements comprise respective abutment protrusions 14 interposed between the extended body and the base wall 2a of the stator 2 and engageable by the releasing member 31.

The peristaltic pump further comprises a thrusting element 20 configured to act, at least when the contrasting elements are in the operative position, in thrust on the contrasting elements 11 in an outwardly direction, wherein the outwardly direction is directed towards the pump race 3 and away from the rotor axis RA. In the embodiment shown in the attached figures, the thrusting element 20 is configured to act in thrust on the contrasting elements 11 both when the contrasting elements are in the operative position and when the contrasting elements are in the released position.

As shown in the embodiment, the pump 1 may comprise one thrusting element 20 active on two or three or more contrasting elements simultaneously. Alternatively, the pump 1 may comprise one thrusting element 20 for each contrasting elements on which the thrusting element 20 is active.

The thrusting element 20 may comprise an elastic element 21, i.e. a spring, a rubber-like element, a compressive spring or a traction spring, acting on the contrasting elements 11 as shown in figures 2-6. In particular one elastic element 21 may be provided for each contrasting element. The elastic element 21 is configured to move the contrasting element 11 in the outwardly direction allowing, and in particular causing, movement of the contrasting elements 11 from the released position to the operative position. Notably the elastic element 21, although it provides a thrust on the contrasting elements 11 directed towards the pump race causing the movement of the contrasting elements 11 from the released position to the operative position, also allows the movement of the contrasting elements 11 from the operative position to the released position.

Notably, the elastic element is configured to exert, when the pump 1 is in the operative configuration, a force on the contrasting elements 11 such that the latter is able to adequately compress, in particular deform, the fluid line 50 to promote fluid flow. Furthermore, the elastic element is also configured to deform itself to allow the at least one contrasting element 11 to move in the opposite direction, namely from the operative position to the released position.

The elastic element may be a compression spring: in this case the compression spring acts on the lever arm 13 at a thrusting seat 15, wherein this thrusting seat 15 may be interposed between the hinged end 13a and the free end 13b of the lever arm 13, defining a third class lever wherein the compression spring defines the applied force and the fluid line defines the resistance force applied on the free end 13b of the contrasting element 11 through a roller. In this third class lever, the thrusting seat 15 is positioned at a distance with respect to the hinge axis HA of the lever arm 13 lower than a corresponding distance between free end 13b carrying the roller 12 and the hinge axis HA.

Alternatively, the free end 13b carrying the roller 12 may be interposed between the hinged end 13a and the thrusting seat 15, defining a second class lever wherein the compression spring defines the applied force and the fluid line defines the resistance force applied on the free end 13b of the contrasting element 11 through a roller. In this second class lever, the thrusting seat 15 is positioned at a distance with respect to the hinge axis HA of the lever arm 13 higher than a corresponding distance between free end 13b carrying the roller 12 and the hinge axis HA.

Alternatively, the elastic element may be a traction spring acting on a pulling portion of the lever arm 13, so that the hinged end 13a is interposed between the pulling portion and the free end 13b carrying the roller, defining a first class lever.

Thus, according to an embodiment wherein the pump comprises the elastic element 21, namely a spring or a rubber-like element, the elastic element is the member in charge of providing a thrusting force, through the contrasting elements 11, on the fluid line 50: this provides a flexible coupling between the rollers 12 and the fluid line, being the elastic element able to eventually compensate variations in the line thickness, stiffness or presence of impurities. Furthermore, the spring rate or the rubber-like element stiffness is set in order to be able to compress the fluid line 50 adequately and determine a fluid flow.

According to a further embodiment, the elastic element may be replaced by a thrusting cam 22 movable by rotation between a thrusting position and a non-thrusting position. In the thrusting position the thrusting cam 22 abuts against the contrasting elements 11 causing the contrasting elements 11 to move in the operative position, while in the non-thrusting position the thrusting cam 22 allows the contrasting element 11 to move in the released position. In addition, the thrusting cam 22, when arranged in the thrusting position, prevents, in particular does not allow, the contrasting elements 11 to move from the operative position to the released position. Notably, in the operative configuration of the pump 1, the thrusting cam 22 is arranged in the thrusting position, while in the released configuration the thrusting cam 22 is arranged in the non-thrusting position.

The peristaltic pump of the present invention also comprises a releasing system 30 comprising a releasing member 31 borne by the stator 2 and movable between an active position and a rest position with respect to the stator 2. In particular the releasing member 31 is engaged to the stator 2 keeping at least one degree of freedom with respect to the stator 2 to shift between the active position and the rest position, i.e. rotation about an own axis or a translation movement. The releasing member 31 of the releasing system 30 is configured, upon movement from the rest position to the active position, to shift the contrasting elements or one of the contrasting elements from the operative position to the released position.

Notably, the releasing member 31 is coupled to the stator 2 and decoupled from the rotor 10. This means that the releasing member 31 does not rotate with the rotor 10 about the rotor axis RA. According to a specific embodiment, the releasing member 31 has only one degree of freedom with respect to the stator 2, namely either rotation or translation.

In particular, during a working condition of the peristaltic pump 1, namely while the rotor 10 rotates about the rotor axis RA, the releasing member 31 is fixed with respect to the stator.

The released position of the contrasting element 11 makes installation and removal procedures of fluid line easier, as the fluid line is not compressed between the pump race 3 and the rollers 12, and also safer, as the fluid line may be removed or installed without forcing it. Furthermore, during time periods wherein the fluid line 50 is installed and the peristaltic pump 1 is not working, the releasing system 30 allows to arrange the constraining element 11 in the released position in order not to stress or damage the fluid line at the contact point. In addition, as also described after in the present description, releasing the contrasting elements 11 allows to re-establish fluid pressure balance between a delivery tract of the fluid line and a supply tract of the fluid line: in other words, this allows to re-establish fluid pressure balance upstream and downstream the peristaltic pump 1.

According to the present invention, the releasing member 31 is spatially shifted with respect to the rotor axis RA and borne by the stator 2: in particular the releasing member 31 emerges in height from the base wall 2a of the stator between the rotor axis RA and the pump race 3 within the internal volume 5 of the stator 2. This feature combination provides benefits to the releasing system of the pump, as the manufacturing process is easier and cheap, and because the releasing member 31 does not affect the rotation of the rotor during a standard working condition of the peristaltic pump. Indeed, in standard working condition, the releasing member 31 is completely decoupled from the rotor movement, thereby improving safety and reliability of the pump.

The peristaltic pump 1 is configurable at least in an operative configuration and a released configuration. In the operative configuration, the releasing member 31 is arranged in the rest position to allow the at least one contrasting element 11 to be arranged in the operative position, and the contrasting element 11 is thrusted by the thrusting element 20 in the outwardly direction towards the pump race 3. On the contrary, in the released configuration, the releasing member 31 is arranged in the active position engaging the contrasting element 11 and moving the contrasting element 11 in the released position away from the pump race 3. Thus, in the operative configuration, the contrasting elements 11 are able to compress the fluid line to cause fluid flow upon rotation of the rotor 10, while in the released configuration the contrasting element 11 is moved away from the pump race, and thus from the fluid line, to allow removal or installation of the fluid line. Notably, in the operative configuration the rotor 10 is configured to rotate about the rotor axis RA to determine a fluid flow: on the contrary, in the released configuration, rotation of the rotor 10 is prevented, in particular the rotation of the rotor is blocked so that the contrasting elements 11 are fixed in position.

In an embodiment not shown in the attached figures, the releasing member 31 may be a translating member movable by translation, i.e. the releasing member may be a lever movable between the active and the rest position, wherein in the active position the lever abuts against one or more of the contrasting elements 11 to move the latter in the released position. The lever may be moved manually by an operator or automatically by an actuator, for example an electric actuator controlled by a control unit 80.

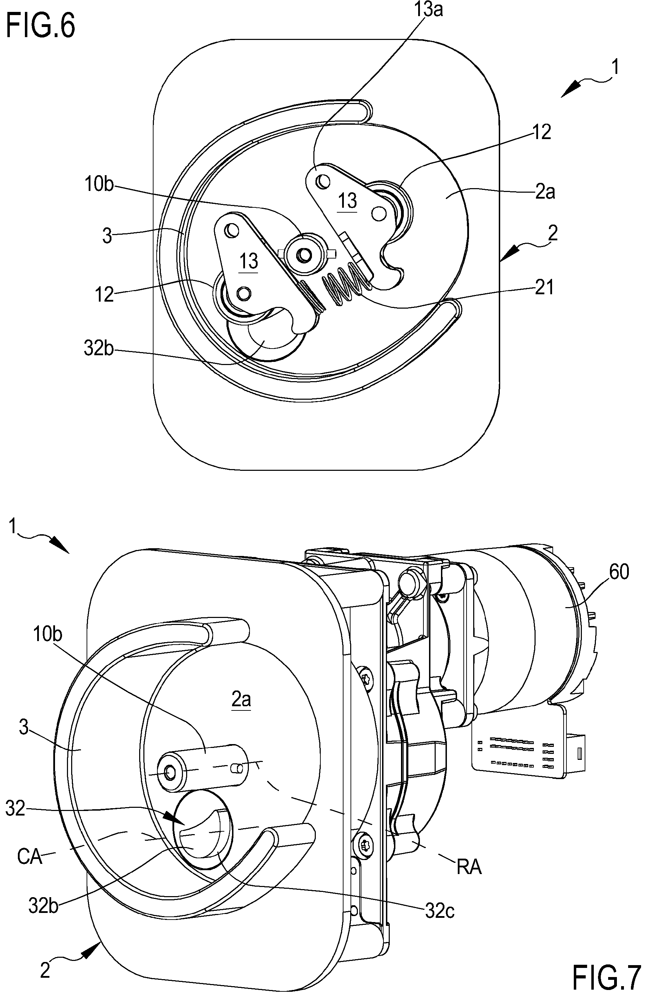

In a further embodiment, the releasing member 31 is a releasing cam 32 rotatable about a cam axis CA: in figure 7 the lever arms 13, the rotor plate 10a and the rollers 12 have been hidden to show in detail the releasing cam 32.

The cam axis CA is spatially shifted with respect to the rotor axis RA: in particular the cam axis CA of the releasing cam 32 is fixed with respect to the stator 2 and the releasing cam 32 is configured to rotate about the cam axis relative to the stator 2. The cam axis CA may be substantially parallel to the rotor axis RA. A distance between the cam axis CA and the rotor axis RA may be comprised between 10mm and 60mm. Anyhow, the distance between the cam axis CA and the rotor axis RA is lower than a distance between the rotor axis RA and the internal wall of the pump race.

Upon rotation of the releasing cam 32, the pump 1 may be configured in the operative configuration or in the released configuration. In more detail, when the pump 1 is in the operative configuration, the releasing cam 32 is rotated in the rest position to allow the at least one contrasting element 11 to move in the operative position: the contrasting element 11 is thrusted by the thrusting element 20 in the outwardly direction. On the contrary, when the pump 1 is in the released configuration, the releasing cam 32 is rotated in the active position, wherein the releasing cam 32 engages the contrasting element 11 and moves the contrasting element 11 in the released position away from the pump race 3.

The releasing cam 32 includes a bearing axle 32a rotatable about the cam axis CA, and a cam-shaped top element 32b fixed at an end of to the bearing axle 32a, as shown in figures 7 and 8. The top element 32b and the bearing axle 32a define the releasing cam 32 as a single body. Furthermore, the base wall 2a of the stator 2 comprises a through hole 4 carrying the bearing axle 32a of the releasing cam 32 as shown in the exploded view of figure 8.

The cam-shaped top element 32b of the releasing cam 32 emerges in height from said base wall 2a of the stator 2 (see figure 7) into the internal volume 5 of the stator defining a cam-shaped lateral abutment wall 32c. This height may be comprised between 1mm and 10mm. The cam-shaped lateral abutment wall 32c defines the thickness of the cam-shaped top element 32b that emerges from to the base wall 2a of the stator, so that the cam-shaped top element 32b is able to cooperate with one or more of the contrasting elements 11. In more detail, when the releasing cam 32 is in the active position, the cam-shaped lateral abutment wall 32c abuts against the at least one contrasting element 11 of the rotor 10, in particular against the abutment portion 14 of the lever arm 13 of the at least one contrasting element 11, to define the released configuration of the peristaltic pump. On the contrary, when the releasing cam 32 is in the rest position, the cam-shaped lateral abutment wall 32c allows the at least one contrasting element 11 to move in the operative position to define the operative configuration of the peristaltic pump.

The releasing member 31 is interposed between the rotor axis RA and the pump race 3 with respect to a radial direction normal to the rotor axis RA. In particular the releasing cam 32, and more in particular the cam axis CA of the releasing cam 32, is interposed between the rotor axis RA and the pump race 3 with respect to a radial direction normal to the rotor axis RA. In addition, the releasing member 31 may be closer to the pump race 3 than to the rotor axis RA.

According to an embodiment, when the peristaltic pump 1 comprises two contrasting elements 11, the releasing member 31, namely the releasing cam 32 or the lever, is configured to engage only one contrasting element at a time of the two or more contrasting elements 11. In other words, the releasing member 31 is configured to act on one single contrasting element 11 at a time, in particular on the contrasting element 11 positioned in the operative arc of the stator 2. The other contrasting element 11 is not engaged by the releasing member and positioned in the non-operative tract of the stator 2, as the two contrasting elements are diametrically opposite each other.

Alternatively, the peristaltic pump 1 may comprise three or more contrasting elements 11: in this case the releasing member 31 is configured to engage either one contrasting element at a time of the three contrasting elements 11, or two contrasting elements at a time of the three contrasting elements 11.

As a general rule, the releasing member 31 may be configured to act only on the contrasting elements 11 which are positioned in the operative arc of the stator 2: indeed, the other contrasting element positioned in the non-operative arc of the stator does not cooperate with the fluid line, making a retraction of these contrasting elements not necessary. In other terms, given at least one first contrasting element positioned in the operative angular arch, this at least one first contrasting element is engageable by the releasing member 31. On the contrary, given at least one second contrasting element positioned in the non-operative angular arch, the at least one second contrasting element is not engageable by the releasing member 31. Thus, the releasing member 31, when passing from the rest position to the active position, is configured to engage the at least one first contrasting element to move the latter in the released position: on the contrary, the releasing member 31, when passing from the rest position to the active position, is configured not to engage the at least one second contrasting element.

In an embodiment according to the preceding description, when switching between the operative configuration and the released configuration, the rotor 10 is arranged in a predefined angular unlocking position: the predefined angular unlocking position is defined as a position wherein at least one of the contrasting elements 11 is engageable by the releasing member 31 to switch from the operative position to the released position and vice versa. More specifically, when the rotor is in the predefined angular unlocking position, only one contrasting element 11 between the two or more contrasting elements 11 is engageable by the releasing member 31.

Notably, when the rotor is in the predefined angular unlocking position, the releasing member 31 is configured to engage all the contrasting elements 11 positioned in the operative arc to move them between the released position and the operative position, while the contrasting elements in the non-operative arc may be not engageable by the releasing member 31.

In the predefined angular unlocking position, the cam-shaped lateral abutment wall 32c of the releasing cam 32 faces the abutment protrusion 14 of the lever arm 13 of one respective contrasting element 11. In particular, the releasing cam 32, when the rotor is in the predefined angular unlocking position, is interposed between the abutment protrusion 14 of the lever arm 13 and the pump race 3 with respect to a radial direction, in particular with respect to a direction normal to the rotor axis RA.

### Drives and control unit

The peristaltic pump 1 may also comprise a drive motor, such as an electric motor, connected to the rotor 10 and configured to cause rotation of the rotor about the rotor axis RA and promote fluid flow within the fluid line 50. Furthermore, a position sensor 70 may be provided and configured to emit a signal representative of an angular position of the rotor 10 with respect to the stator 2. In particular the position sensor 70 of the rotor is a Hall sensor configured to detect the position of a roller or shoe 12 or a passage of a roller or shoe while the rotor 10 rotates.

In addition, an actuator, i.e. an electric actuator or motor, may be provided and connected to the releasing member 31 and configured to move the releasing member between the active position and the rest position. In particular, in case the releasing cam 32 is provided, the actuator is configured to rotate the releasing cam 32 between the active position and the rest position. Furthermore, a position sensor 71 may be provided and configured to emit a signal representative of the active position or the rest position of the releasing member 31.

The peristaltic pump may comprise a control unit 80 connected to at least one between the drive member 40, the position sensor 70 of the rotor 10, the actuator of the releasing member 31 and the position sensor of the releasing member 31.

When the control unit is connected at least to the drive member 40 and the position sensor 70 of the rotor 10, the control unit 80 is configured to receive the signal emitted by the position sensor 70 of the rotor 10, and to determine an angular position of the rotor with respect to the stator 2.

Analogously, when the control unit is connected at least to the actuator of the releasing member 31 and the corresponding position sensor 71 of the releasing member 31, the control unit 80 is configured to receive the signal emitted by the position sensor of the releasing member 31, and selectively determine whether the releasing member is in the active position or in the rest position.

The control unit may be configured to switch the peristaltic pump 1 from the operative configuration to the released configuration by performing at least the following steps:
- arresting or rotating the rotor 10 at a predefined angular unlocking position with respect to the stator 2, the predefined angular unlocking position being defined as a position wherein at least one of the contrasting elements 11 is engageable by the releasing member 31 to switch from the operative position to the released position and vice versa;
- moving the releasing member 31, in particular rotating a releasing cam 32, from the rest position to the active position to move the at least one contrasting element 11 from the operative position to the released position, said step of moving the releasing member 31 being performed manually by an operator or by a control unit 80 through an actuator;
and wherein the control unit 80 is configured to switch the peristaltic pump 1 from the released configuration to the operative configuration by performing at least the following steps:
- arresting or rotating the rotor 10 at a predefined angular unlocking position with respect to the stator 2, the predefined angular unlocking position being defined as a position wherein at least one of the contrasting elements 11 is engageable by the releasing member 31 to switch from the operative position to the released position and vice versa;
- moving the releasing member 31, in particular rotating the releasing cam 32, from the active position to the rest position to move the at least one contrasting element 11 from the released position to the operative position, said step of moving the releasing member 31 being performed manually by an operator or by the control unit 80 through an actuator.

The control unit may be also configured to perform a substituting procedure to replace a fluid line 50 in the peristaltic pump 1 by switching the pump from the operative configuration to the released configuration, removing the old fluid line, installing a new fluid line, and subsequently switching the pump from the released configuration to the operative configuration. In particular the substituting procedure comprises at least the steps of:
- arresting, or rotating the rotor 10, at the predefined angular unlocking position based on the position detected by the position sensor 70 of the rotor 10;
- moving the releasing member 31, in particular rotating the releasing cam 32, from the rest position to the active position to move the at least one contrasting element 11 from the operative position to the released position, this step of moving the releasing member 31 may be performed manually by an operator or by the control unit 80 through the actuator of the releasing member 31;
- removing the fluid line 50 between the at least one contrasting element 11 and the pump race 3, in particular removing the fluid line 50 between the contrasting element 11 in the operative arc and the pump race 3;
- installing the fluid line 50 between the at least one contrasting element 11 and the pump race 3;
- moving the releasing member 31, in particular rotating the releasing cam 32, from the active position to the rest position to move the at least one contrasting element 11 from the released position to the operative position, said step of moving the releasing member 31 being performed manually by an operator or by the control unit 80 through an actuator.

The control unit 80 may also be configured to perform a releasing procedure and an installing procedure, wherein the releasing procedure is configured to remove the fluid line from the peristaltic pump 1, and the installing procedure is configured to install the fluid line in the peristaltic pump 1. Combination of the releasing procedure with the installing procedure results in the substituting procedure.

The releasing procedure comprises at least the steps of:
- arresting, or rotating the rotor 10, at the predefined angular unlocking position based on the position detected by the position sensor 70 of the rotor 10;
- moving the releasing member 31, in particular rotating the releasing cam 32, from the rest position to the active position to move the at least one contrasting element 11 from the operative position to the released position, this step of moving the releasing member 31 may be performed manually by an operator or by the control unit 80 through the actuator of the releasing member 31;
- removing the fluid line 50 between the at least one contrasting element 11 and the pump race 3, in particular removing the fluid line 50 between the contrasting element 11 in the operative arc and the pump race 3.

The setting procedure comprises at least the steps of:
- arresting, or rotating the rotor 10, at the predefined angular unlocking position based on the position detected by the position sensor 70 of the rotor 10;
- moving the releasing member 31, in particular rotating the releasing cam 32, from the rest position to the active position to move the at least one contrasting element 11 from the operative position to the released position, this step of moving the releasing member 31 may be performed manually by an operator or by the control unit 80 through the actuator of the releasing member 31;
- installing the fluid line 50 between the at least one contrasting element 11 and the pump race 3;
- moving the releasing member 31, in particular rotating the releasing cam 32, from the active position to the rest position to move the at least one contrasting element 11 from the released position to the operative position, said step of moving the releasing member 31 being performed manually by an operator or by the control unit 80 through an actuator.

Notably, the step of arresting or rotating the rotor 10, in the setting, releasing and/or substituting procedure, may comprise the step of commanding the drive motor 60 to position the rotor 10 at the predefined angular unlocking position.

As already mentioned in description, the peristaltic pump of the present invention may allow to balance fluid pressures downstream and upstream the peristaltic pump 1 or periodically lower a differential pressure between downstream and upstream sections of the fluid line with respect to the peristaltic pump 1.

A contrasting element 11 in the operative position abuts against the fluid line at a contact point causing the fluid line to be compressed, thereby limiting or inhibiting fluid communication across the contact point between fluid located upstream the pump and fluid located downstream the pump. Shifting the contrasting element 11 from its operative position to released position allows the fluid line 50 to re-expand at contact point, causing the fluid located upstream the pump 1 to be in fluid communication with the fluid located downstream the pump 1, thereby eventually rebalancing a differential pressure.

The control unit 80 is configured to perform a pressure balancing procedure to re-balance fluid pressures across the pump, wherein the pressure balancing procedure comprises switching the pump from the operative configuration to the released configuration. In particular the balancing procedure may comprise the steps of:
- arresting, or rotating the rotor 10, at the predefined angular unlocking position based on the position detected by the position sensor 70 of the rotor 10;
- moving the releasing member 31, in particular rotating the releasing cam 32, from the rest position to the active position to move the at least one contrasting element 11 from the operative position to the released position, this step of moving the releasing member 31 may be performed manually by an operator or by the control unit 80 through the actuator of the releasing member 31.

Furthermore, the control unit 80 may be configured to:
- receive at least a pressure signal representative of a fluid pressure upstream and downstream the peristaltic pump 1 or a signal representative of a differential pressure across the pump, in particular a differential pressure between an upstream section of the fluid line and a downstream section of the fluid line;
- determine a differential pressure value representative of a differential pressure across the pump 1;
- compare the differential pressure value with at least one respective threshold value;
- if the differential pressure value exceeds the at least one respective threshold value, perform the balancing procedure.

According to an embodiment, the balancing procedure is performed during an extracorporeal blood treatment, in particular wherein the fluid in the fluid line 50 is blood.

As already mentioned in the description, a prolonged compression of one roller 12 on the fluid line during a period of inactivity of the pump 1, namely during a period wherein the rotor 10 does not rotate, may lead to damage the fluid line at the contact point with the roller. For example, a prolonged compression may lead to plastic deformation of the fluid line.

In order to avoid this issue, the pump 1 of the present invention allows to retract the roller during inactivity periods of the pump 1, so that no compression or a reduced compression is applied on the fluid line by the roller. On this regard, the control unit 80 is configured to perform a inactivity procedure to prevent damage to the fluid line 50 during prolonged inactivity periods of the pump 1. The inactivity procedure comprises switching the pump from the operative configuration to the released configuration. In particular the inactivity procedure comprises the steps of:
- arresting or rotating the rotor 10 at the predefined angular unlocking position based on the position detected by the position sensor 70;
- moving the releasing member 31, in particular rotating the releasing cam 32, from the rest position to the active position to move the at least one contrasting element 11 from the operative position to the released position, said step of moving the releasing member 31 being performed manually by an operator or by the control unit 80 through an actuator.

During the prolonged inactivity periods of the pump 1, the fluid line 50 is mounted on the pump 1 interposed between the contrasting elements 11 and the pump race 3.

The inactivity procedure may be triggered by an operator through a user interface, such as a graphical user interface or a button, operatively connected to the control unit 80. Alternatively, the control unit may be configured to:
- evaluate an inactivity time period within which the pump is inactive;
- compare said inactivity time period with a respective inactivity time period threshold;
- if the inactivity time period exceeds the inactivity time period threshold, trigger the inactivity procedure.

The threshold period may be received as input by a user by a user interface or may be a predefined value. The inactivity time period threshold may be comprised between 2 minutes and 1 hour, in particular between 5 minutes and 30 minutes.

The control unit may be also configured to perform safety procedures aimed to prevent damages to the pump and increase reliability of the pump. According to this aim, the control unit 80 may be configured to:
- detect the position of the rotor 10 by the position sensor 70;
- evaluate whether said detected position of the rotor 10 is coincident with the predefined angular unlocking position;
- if the detected position of the rotor 10 is coincident with the predefined angular unlocking position, allow the actuator of the releasing member 31 to move the releasing member 31 from the rest position to the active position;
- if the detected position of the rotor 10 does not coincide with the predefined angular unlocking position, prevent the actuator of the releasing member 31 to move the releasing member 31 from the rest position to the active position.

Furthermore, the control unit 80 may be configured to:
- detect the position of the releasing member 31 by the position sensor 71 of the releasing system 30;
- evaluate whether this detected position of the releasing member 31 corresponds to the active position or the rest position;
- if the releasing member 31 is detected to be in the active position, prevent rotation of the rotor 10 by inhibiting rotation of the drive motor 60 of the rotor 10.

The step of preventing rotation of the drive motor 60 may be performed by the control unit by preventing the drive motor 60 to be supplied by an electrical current.

As a general, a rotation of the rotor 10, when the pump 1 is in the released configuration, is prevented, in particular blocked. On the other hand, when the pump 1 is in the operative configuration, a rotation of the rotor 10 is allowed.

### Methods

The present disclosure is also directed to methods performed by the peristatic pump 1 according to the preceding description and to the attached claims.

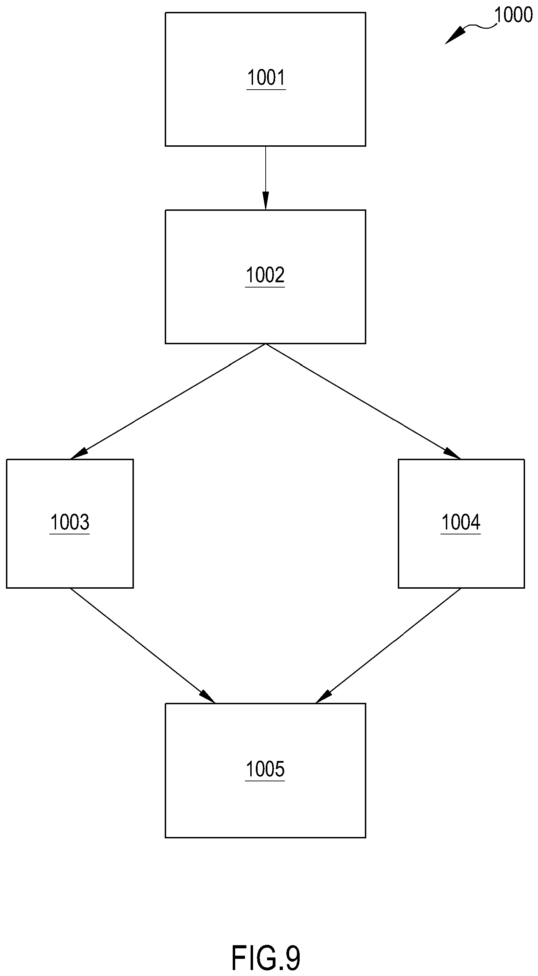

A method 1000, shown in the flow chart of figure 9, is provided to switch the peristaltic pump 1 from the operative configuration to the released configuration and vice versa. The method to switch the peristaltic pump 1 from the operative configuration to the released configuration comprises:
- a step 1001 including arresting or rotating the rotor 10 at a predefined angular unlocking position with respect to the stator 2, the predefined angular unlocking position being defined as a position wherein at least one of the contrasting elements 11 is engageable by the releasing member 31 to switch from the operative position to the released position and vice versa;
- a step 1002 including moving the releasing member 31, in particular rotating a releasing cam 32, from the rest position to the active position to move the at least one contrasting element 11 from the operative position to the released position, said step of moving the releasing member 31 being performed manually by an operator or by a control unit 80 through an actuator

The method to switch the peristaltic pump 1 from the released configuration to the operative configuration comprises at least:
- arresting or rotating the rotor 10 at a predefined angular unlocking position with respect to the stator 2, the predefined angular unlocking position being defined as a position wherein at least one of the contrasting elements 11 is engageable by the releasing member 31 to switch from the operative position to the released position and vice versa;
- a step 1005 including moving the releasing member 31, in particular rotating the releasing cam 32, from the active position to the rest position to move the at least one contrasting element 11 from the released position to the operative position, said step of moving the releasing member 31 being performed manually by an operator or by the control unit 80 through an actuator.

In addition, the method may comprise:
- a step 1003 including removing the fluid line 50 from the peristaltic pump 1 when the pump is in the released configuration, and/or
- a step 1004 including installing the fluid line 50 in the peristaltic pump 1 when the pump is in the released configuration.

Notably, the step of switching the pump from the operative configuration to the released configuration causes balancing of fluid pressures across the pump, in particular wherein a fluid differential pressure between upstream and downstream tracts of the fluid line during an operative configuration is balanced. In particular the differential pressure is reduced or reset to zero when the pump is arranged in the released configuration as the fluid upstream the pump is in fluid communication with the fluid downstream the pump.

The method may also comprise an inactivity procedure to prevent damages to a fluid line 50 installed on the pump 1 during a prolonged period of inactivity of the pump 1: the inactivity procedure comprises a step of switching the pump 1 from the operative configuration to the released configuration or keeping the pump in the released configuration. This allows to remove compression provided by the roller 12 on the fluid line when the contrasting element 11 is in the operative position, namely when the roller squeezes the fluid line.

Furthermore, the inactivity procedure may comprise:
- evaluating an inactivity time period within which the pump (1) is inactive;
- comparing said inactivity time period with a inactivity time period threshold;
- if the inactivity time period exceeds the inactivity time period threshold, switching the pump (1) from the operative configuration to the released configuration or keeping the pump in the released configuration.

The inactivity time period threshold may be comprised between 2 minutes and 1 hour, in particular between 5 minutes and 30 minutes.

A method for removing a fluid line 50 in a peristaltic pump 1 may also be provided, wherein this method comprises a releasing procedure to switch the peristaltic pump 1 from the operative configuration to the released configuration, the releasing procedure comprising at least the following steps:
- arresting or rotating the rotor 10 at a predefined angular unlocking position with respect to the stator 2, the predefined angular unlocking position being defined as a position wherein at least one of the contrasting elements 11 is engageable by the releasing member 31 to switch from the operative position to the released position and vice versa;
- moving the releasing member 31, in particular rotating a releasing cam 32, from the rest position to the active position to move the at least one contrasting element 11 from the operative position to the released position, said step of moving the releasing member 31 being performed manually by an operator or by a control unit 80 through an actuator;
- removing the fluid line 50 from the pump 1 or installing the fluid line 50 between the at least one contrasting element 11 and the pump race 3.

A method for installing a fluid line 50 in a peristaltic pump 1 is provided, the setting procedure comprising at least the following steps:
- arresting or rotating the rotor 10 at a predefined angular unlocking position based on the position detected by the position sensor 70;
- evaluating whether the releasing member 31 is in the rest position or in the active position, if releasing member 31 is in the rest position moving the releasing member 31, in particular rotating the releasing cam 32, from the rest position to the active position to move the at least one contrasting element 11 from the operative position to the released position, said step of moving the releasing member 31 being performed manually by an operator or by the control unit 80 through an actuator;
- installing the fluid line 50 between the at least one contrasting element 11 and the pump race 3
- moving the releasing member 31, in particular rotating the releasing cam 32, from the active position to the rest position to move the at least one contrasting element 11 from the released position to the operative position, said step of moving the releasing member 31 being performed manually by an operator or by the control unit 80 through an actuator.

A method for replacing a flud line 50 is a peristaltic pump 1 may be also provided, wherein the method comprises a substituting procedure including the steps of:
- arresting or rotating the rotor 10 at a predefined angular unlocking position based on the position detected by the position sensor 70;
- moving the releasing member 31, in particular rotating the releasing cam 32, from the rest position to the active position to move the at least one contrasting element 11 from the operative position to the released position, said step of moving the releasing member 31 being performed manually by an operator or by the control unit 80 through an actuator;
- removing the fluid line 50 between the at least one contrasting element 11 and the pump race 3;
- installing the fluid line 50 between the at least one contrasting element 11 and the pump race 3
- moving the releasing member 31, in particular rotating the releasing cam 32, from the active position to the rest position to move the at least one contrasting element 11 from the released position to the operative position, said step of moving the releasing member 31 being performed manually by an operator or by the control unit 80 through an actuator.

A method for preventing damage to a fluid line 50 during prolonged inactivity periods of a peristaltic pump 1 may be provided, wherein this method comprises an inactivity procedure including at least the steps of:
- arresting or rotating the rotor 10 at the predefined angular unlocking position based on the position detected by the position sensor 70;
- moving the releasing member 31, in particular rotating the releasing cam 32, from the rest position to the active position to move the at least one contrasting element 11 from the operative position to the released position, said step of moving the releasing member 31 being performed manually by an operator or by the control unit 80 through an actuator;
wherein, during said prolonged inactivity periods of the pump 1, the fluid line 50 is mounted on the pump 1, in particular wherein the fluid line 50 is interposed between at least one of the contrasting element 11 and the pump race 3.

A method for balancing fluid pressures across a peristaltic pump 1 may be also provide, wherein the method comprises a pressure balancing procedure including at least the steps of:
- arresting, or rotating the rotor 10, at the predefined angular unlocking position based on the position detected by the position sensor 70 of the rotor 10;
- moving the releasing member 31, in particular rotating the releasing cam 32, from the rest position to the active position to move the at least one contrasting element 11 from the operative position to the released position, this step of moving the releasing member 31 being performed manually by an operator or by the control unit 80 through the actuator of the releasing member 31.

The balancing procedure of the previous method may also comprise the steps of:
- receiving at least one pressure signal representative of a fluid pressure upstream and downstream the peristaltic pump 1 or a signal representative of a differential pressure across the pump 1, in particular a differential pressure between an upstream section of the fluid line and a downstream section of the fluid line;
- determining a differential pressure value representative of a differential pressure across the pump 1;
- comparing said differential pressure value with at least one respective threshold value;
- if said differential pressure value exceeds the at least one respective threshold value, performing the balancing procedure.

## Claims

1. Peristaltic pump (1) configured to receive a fluid line (50), said peristaltic pump comprising:
- a stator (2) comprising a pump race (3);
- a rotor (10) surrounded at least partially by the pump race (3) and configured to rotate about a rotor axis (RA), said rotor (10) comprising:
o at least one contrasting element (11) configured to abut against the fluid line (50), the at least one contrasting element (11) being movable towards and away with respect to the rotor axis (RA) and the pump race (3) between:
▪ a released position wherein the at least one contrasting element (11) is away from the pump race (3), and
▪ an operative position wherein the at least one contrasting element (11) is approached to the pump race (3) and configured to abut against the fluid line (50), in the released position the at least one contrasting element (11) being at a distance from the pump race (3) higher than in the operative position,
o a thrusting element (20) configured to act, at least in the operative position, in thrust on said contrasting element (11) in an outwardly direction towards the pump race (3); and
- a releasing system (30) comprising a releasing member (31) movable between an active position and a rest position;
wherein the peristaltic pump (1) is configurable in at least:
- an operative configuration wherein the releasing member (31) is moved in the rest position to allow the at least one contrasting element (11) to move in the operative position, the contrasting element (11) being thrusted by the thrusting element (20) in the outwardly direction; and
- a released configuration wherein the releasing member (31) is moved in the active position, said releasing member (31) engaging the contrasting element (11) and moving the contrasting element (11) in the released position away from the pump race (3);
**characterized in that** the releasing member (31) is borne by the stator (2) and is spatially shifted with respect to the rotor axis (RA).

2. Peristaltic pump according to the preceding claim, wherein:
- the releasing member (31) is a releasing cam (32) rotatable about a cam axis (CA), said cam axis being spatially shifted with respect to the rotor axis (RA), and wherein:
o in the operative configuration, the releasing cam (32) is rotated in the rest position to allow the at least one contrasting element (11) to remain in the operative position, the contrasting element (11) being thrusted by the thrusting element (20) in the outwardly direction;
o in the released configuration, the releasing cam (32) is rotated in the active position, said releasing cam (32) engaging the at least one contrasting element (11) and maintaining the at least one contrasting element (11) in the released position away from the pump race (3);
or
- the releasing member (31) is a translating member movable by translation along a rectilinear or curvilinear path, wherein:
o in the operative configuration, the translating member is moved in the rest position to allow the at least one contrasting element (11) to remain in the operative position, the contrasting element (11) being thrusted by the thrusting element (20) in the outwardly direction;
o in the released configuration, the translating member is moved in the active position, said translating member engaging the at least one contrasting element (11) and maintaining the at least one contrasting element (11) in the released position away from the pump race (3).

3. Peristaltic pump according to the preceding claim, wherein the releasing member (31) is the releasing cam (32),
the cam axis (CA) of the releasing cam (32) being fixed with respect to the stator (2) and wherein the releasing cam (32) is configured to rotate about the cam axis relative to the stator (2),
in particular wherein the cam axis (CA) is substantially parallel to the rotor axis (RA).

4. Peristaltic pump according to anyone of the preceding claims, wherein the releasing member (31), in particular the releasing cam (32) and more in particular the cam axis (CA) of the releasing cam (32), is interposed between the rotor axis (RA) and the pump race (3) with respect to a radial direction normal to the rotor axis (RA).

5. Peristaltic pump according to anyone of the preceding claims, wherein the stator (2) comprises a base wall (2a), the pump race (3) protruding in height from said base wall (2a) defining an internal volume confined in depth by the base wall (2a) and laterally by the pump race (3), the rotor (10) being arranged at least partially within said internal volume of the stator (2),
wherein the releasing cam (32) includes a bearing axle (32a) rotatable about the cam axis (CA), and a cam-shaped top element (32b) fixed at an end of to the bearing axle (32a), the bearing axle (32a) in combination with the cam-shaped top element (32b) defining the releasing cam (32) as a single body,
and wherein the base wall (2a) of the stator (2) comprises a through hole (4) allowing the bearing axle (32a) of the releasing cam (32) to pass through, the cam-shaped top element (32b) of the releasing cam (32) emerging in height from said base wall (2a) of the stator (2) into the internal volume defining a cam-shaped lateral abutment wall (32c),
wherein:
- when the releasing cam (32) is in the active position, said cam-shaped lateral abutment wall (32c) abuts against the at least one contrasting element (11) of the rotor (10) to define the released configuration of the peristaltic pump, and
- when the releasing cam (32) is in the rest position, said cam-shaped lateral abutment wall (32c) allows the at least one contrasting element (11) to remain in the operative position to define the operative configuration of the peristaltic pump.

6. Peristaltic pump according to any one of the preceding claims, wherein the rotor (10) comprises a rotor plate (10a) bearing the at least one contrasting element (11), and wherein each contrasting element (11) includes:
- at least one roller or shoe (12) configured to contact and squeeze the fluid line (50) against the pump race (3) in the operative condition of the peristaltic pump;
- a lever arm (13) extending between a hinged end (13a) and a free end (13b), said lever arm (13) being rotatable about the hinged end (13a) about a hinge axis (HA), wherein:
o the hinged end (13a) is hinged to said rotor plate (10a) thereby allowing rotation of the lever arm (13) about the hinge axis (HA), and
o the free end (13b) carries the at least one roller or shoe (12),
wherein a rotation of the lever arm (13) about the hinge axis (HA) determines the passage of the respective contrasting element (11) between the operative position and the released position, in particular wherein the distance between the contrasting element and the pump race (3) is defined as a distance interposed between the roller or shoe (12) and the pump race (3).

7. Peristaltic pump according to the preceding claim, wherein the lever arm (13) comprises an extended body and an abutment protrusion (14) emerging from said extended body, said abutment protrusion (14) being configured to abut against the cam-shaped lateral abutment wall (32c) of the releasing cam (32) when the releasing cam is in the active position, said abutment protrusion (14) being interposed between said extended body and the base wall (2a) of the stator (2).

8. Peristaltic pump according to any one of the preceding claims, wherein the pump (1) comprises:
- two or more contrasting elements (11) substantially diametrically opposite each other with respect to the rotor axis (RA), the releasing member (31) being configured to engage one contrasting element at a time between the two or more contrasting elements (11), or
- three or more contrasting elements (11) angularly spaced each other in a substantially evenly manner, in particular the three or more contrasting elements being equally spaced each other, the releasing member (31) being configured to engage one or two contrasting elements at a time between the three or more contrasting elements (11).

9. Peristaltic pump according to any one of the preceding claims, wherein the thrusting element (20) comprises:
- at least one elastic element (21), in particular a spring or a rubber-like element, more in particular a compressive or traction spring, acting on the at least one contrasting element (11), in particular on each contrasting element, and configured to move the at least one contrasting element (11) in the outwardly direction, said at least one elastic element causing movement of the at least one contrasting element (11) from the released position to the operative position, and wherein the releasing member (31), in particular the releasing cam (32), is configured, during a passage between the operative configuration to the released configuration, to exert a force on the at least one contrasting element (11) opposite in direction to a force exerted by the thrusting element (20) on the at least one contrasting element (11);
or
- a thrusting cam (22) movable by rotation between:
o a thrusting position wherein the thrusting cam (22) abuts against the at least one contrasting element (11) causing the contrasting element (11) to move in the operative position, and
o a non-thrusting position wherein the thrusting cam (22) allows the contrasting element (11) to move in the released position.

10. Peristaltic pump according to the preceding claim, wherein the elastic element is configured to:
- exert, when the pump is in the operative configuration, a force on the at least one contrasting element (11) such that the latter is able to adequately squeeze, in particular deform, the fluid line (50) to promote fluid flow, and
- deform itself to allow the at least one contrasting element (11) to move from the operative position to the released position.

11. Peristaltic pump according to any one of the preceding claims, wherein, when switching between the operative configuration and the released configuration, the rotor (10) is arranged in a predefined angular unlocking position,
wherein, when the rotor (10) is in said predefined angular unlocking position, at least one of the contrasting elements (11) is engageable by the releasing member (31) to switch from the operative position to the released position and vice versa,
the pump race (3) having:
- an inner wall configured to contact the fluid line, said inner wall defining an operative angular arch for the contrasting elements, said contrasting elements facing said inner wall of the pump race, and
- a lateral aperture configured to allow an inlet tract of the fluid line (50) to be received by the pump, and an exit tract of the fluid lined to exit the pump, said lateral aperture defining a non-operative angular arch for the contrasting elements, in particular the contrasting elements when in said non-operative angular arch are not configured to fully cooperate with the fluid line (50),
and wherein the rotor (10), when arranged in the predefined angular unlocking position, has:
- at least one first contrasting element positioned in the operative angular arch, wherein said at least one first contrasting element is engageable by the releasing member (31); and
- at least one second contrasting element positioned in said non-operative angular arch, wherein said at least one second contrasting element is not engageable by the releasing member (31),
wherein the releasing member 31, when arranged in the predefined angular unlocking position, is configured to engage said at least one first contrasting element to move the latter in the released position,
in particular wherein the releasing member 31, when passing from the rest position to the active position, is configured not to engage said at least one second contrasting element.

12. Peristaltic pump according to the preceding claim, wherein the peristaltic pump comprises:
- a drive motor (60) operatively connected to the rotor (10) and configured to rotate said rotor about the rotor axis (RA) to determine a fluid flow in the fluid line (50);
- a position sensor (70) configured to emit a signal representative of an angular position of the rotor (10) with respect to the stator (2), said position sensor being in particular a Hall sensor configured to detect a position or a passage of the roller or shoe (12);
- a control unit (80) operatively connected to the drive motor (60) and the position sensor (70), the control unit being configured to receive the signal emitted by the position sensor (70) and to determine an angular position of the rotor with respect to the stator (2) or a position of at least one contrasting element (11) with respect to the releasing member (31),
and wherein the control unit is configured to perform a:
- releasing procedure configured to switch the peristaltic pump from the operative configuration to the released configuration, said releasing procedure comprising:
o arresting or rotating the rotor (10) to the predefined angular unlocking position based on the position detected by the position sensor (70);
o moving the releasing member (31), in particular rotating the releasing cam (32), from the rest position to the active position to move the at least one contrasting element (11) from the operative position to the released position, said step of moving the releasing member (31) being performed manually by an operator or by the control unit (80) through an actuator;
o removing the fluid line (50) between the at least one contrasting element (11) and the pump race (3);
and
- a setting procedure, subsequent to the releasing procedure, configured to switch the peristaltic pump from the released configuration to the operative configuration, said setting procedure comprising:
o arresting or rotating the rotor (10) to the predefined angular unlocking position based on the position detected by the position sensor (70);
o moving the releasing member (31), in particular rotating the releasing cam (32), from the rest position to the active position to move the at least one contrasting element (11) from the operative position to the released position, said step of moving the releasing member (31) being performed manually by an operator or by the control unit (80) through an actuator;
o installing the fluid line (50) between the at least one contrasting element (11) and the pump race (3)
o moving the releasing member (31), in particular rotating the releasing cam (32), from the active position to the rest position to move the at least one contrasting element (11) from the released position to the operative position, said step of moving the releasing member (31) being performed manually by an operator or by the control unit (80) through an actuator,
in particular wherein the steps of installing and removing the fluid line (50) from the peristaltic pump (1) are performed by a user.

13. Peristaltic pump according to any one of the preceding claims, wherein the releasing system (30) comprises a position sensor (71) operatively connected to the control unit (80) and configured to emit a signal representative of the active or rest position of the releasing member (31), said control unit (80) being configured to selectively determine whether the releasing member (31) is in the active or rest position,
the pump further comprising an actuator operatively connected to the control unit (80) and to the releasing member (31), the actuator being configured to move the releasing member (31) between the rest position and the active position upon command of the control unit (80),
and wherein the control unit (80) is configured to:
- detect the position of the releasing member (31) by the position sensor (71) of the releasing system (30);
- evaluate whether said detected position of the releasing member (31) corresponds to the active position or the rest position;
- if the releasing member (31) is detected to be in the active position, prevent rotation of the rotor (10) by inhibiting rotation of the drive motor (60) of the rotor (10).

14. Peristaltic pump according to any one of the preceding claims, wherein the distance between the at least one contrasting element (11) and the pump race (3) is defined as a distance interposed between a portion of the contrasting element configured to abut against the fluid line (50) and the pump race (3),
in particular wherein the distance between the at least one contrasting element (11) and the pump race (3) is defined as a distance interposed between a roller or shoe (12), when arranged in the operative arc of the pump race (3), and the pump race (3),
and wherein the releasing member (31) does not rotate with the rotor (10) about the rotor axis (RA), and wherein, during a working condition of the peristaltic pump (1), in particular during a rotation of the rotor (10) about the rotor axis (RA), the releasing member (21) is fixed with respect to the stator (2) and the rotor (10) is configured to rotate with respect to the releasing member (31).

15. Method (1000) to switch the peristaltic pump (1) from the operative configuration to the released configuration and vice versa, said peristaltic pump (1) being according to any one of the preceding claims,
wherein the method to switch the peristaltic pump (1) from the operative configuration to the released configuration comprises at least the following steps:
- arresting or rotating the rotor (10) to a predefined angular unlocking position with respect to the stator (2), the predefined angular unlocking position being defined as a position wherein at least one of the contrasting elements (11) is engageable by the releasing member (31) to switch from the operative position to the released position and vice versa;
- moving the releasing member (31), in particular rotating a releasing cam (32), from the rest position to the active position to move the at least one contrasting element (11) from the operative position to the released position, said step of moving the releasing member (31) being performed manually by an operator or by a control unit (80) through an actuator;
and wherein the method to switch the peristaltic pump (1) from the released configuration to the operative configuration comprises at least the following steps:
- arresting or rotating the rotor (10) to a predefined angular unlocking position with respect to the stator (2), the predefined angular unlocking position being defined as a position wherein at least one of the contrasting elements (11) is engageable by the releasing member (31) to switch from the operative position to the released position and vice versa;
- moving the releasing member (31), in particular rotating the releasing cam (32), from the active position to the rest position to move the at least one contrasting element (11) from the released position to the operative position, said step of moving the releasing member (31) being performed manually by an operator or by the control unit (80) through an actuator.

## Patentansprüche

1. Peristaltische Pumpe (1), die dazu konfiguriert ist, einen Schlauch (50) aufzunehmen, wobei die peristaltische Pumpe Folgendes umfasst:
- einen Stator (2), der einen Pumpenlaufring (3) umfasst;
- einen Rotor (10), der wenigstens teilweise von dem Pumpenlaufring (3) umgeben und dazu konfiguriert ist, sich um eine Rotorachse (RA) zu drehen, wobei der Rotor (10) Folgendes umfasst:
o wenigstens ein gegensätzliches Element (11), das dazu konfiguriert ist, an dem Schlauch (50) anzuliegen, wobei das wenigstens eine gegensätzliche Element (11) in Bezug auf die Rotorachse (RA) und den Pumpenlaufring (3) hin- und wegbewegbar ist zwischen:
▪ einer gelösten Position, in der das wenigstens eine gegensätzliche Element (11) weg von dem Pumpenlaufring (3) befindlich ist, und
▪ einer Betriebsposition, in der das wenigstens eine gegensätzliche Element (11) an den Pumpenlaufring (3) angenähert und dazu konfiguriert ist, an dem Schlauch (50) anzuliegen, wobei in der gelösten Position das wenigstens eine gegensätzliche Element (11) sich in einem Abstand von dem Pumpenlaufring (3) befindet, der größer ist als in der Betriebsposition,
o ein Schubelement (20), das dazu konfiguriert ist, wenigstens in der Betriebsposition Schub auf das gegensätzliche Element (11) in einer Auswärtsrichtung hin zu dem Pumpenlaufring (3) auszuüben; und
- ein Lösesystem (30), das ein Löseelement (31) umfasst, das zwischen einer aktiven Position und einer Ruheposition bewegbar ist;
wobei die peristaltische Pumpe (1) wenigstens in Folgendes konfigurierbar ist:
- eine Betriebskonfiguration, in der das Löseelement (31) in die Ruheposition bewegt ist, damit das wenigstens eine gegensätzliche Element (11) sich in die Betriebsposition bewegen kann, wobei das gegensätzliche Element (11) durch das Schubelement (20) in die Auswärtsrichtung geschoben wird; und
- eine gelöste Konfiguration, in der das Löseelement (31) in die aktive Position bewegt ist, wobei das Löseelement (31) das gegensätzliche Element (11) in Eingriff nimmt und das gegensätzliche Element (11) in die gelöste Position, weg von dem Pumpenlaufring (3), bewegt;
**dadurch gekennzeichnet, dass** das Löseelement (31) von dem Stator (2) getragen wird und in Bezug auf die Rotorachse (RA) räumlich versetzt ist.

2. Peristaltische Pumpe nach dem vorangehenden Anspruch, wobei:
- das Löseelement (31) ein Lösenocken (32) ist, der um eine Nockenachse (CA) drehbar ist, wobei die Nockenachse in Bezug auf die Rotorachse (RA) räumlich versetzt ist, und wobei:
o in der Betriebskonfiguration der Lösenocken (32) in die Ruheposition gedreht ist, damit das wenigstens eine gegensätzliche Element (11) in der Betriebsposition bleiben kann, wobei das gegensätzliche Element (11) durch das Schubelement (20) in die Auswärtsrichtung geschoben wird;
o in der gelösten Konfiguration der Lösenocken (32) in die aktive Position gedreht ist, wobei der Lösenocken (32) das wenigstens eine gegensätzliche Element (11) in Eingriff nimmt und das wenigstens eine gegensätzliche Element (11) in der gelösten Position, weg von dem Pumpenlaufring (3), hält;
oder
- das Löseelement (31) ein sich verlagerndes Element ist, das durch Translation entlang eines geradlinigen oder krummlinigen Weges bewegbar ist, wobei:
o in der Betriebskonfiguration das sich verlagernde Element in die Ruheposition bewegt ist, damit das wenigstens eine gegensätzliche Element (11) in der Betriebsposition bleiben kann, wobei das gegensätzliche Element (11) durch das Schubelement (20) in die Auswärtsrichtung geschoben wird;
o in der gelösten Konfiguration das sich verlagernde Element in die aktive Position bewegt ist, wobei das sich verlagernde Element das wenigstens eine gegensätzliche Element (11) in Eingriff nimmt und das wenigstens eine gegensätzliche Element (11) in der gelösten Position, weg von dem Pumpenlaufring (3), hält.

3. Peristaltische Pumpe nach dem vorangehenden Anspruch, wobei das Löseelement (31) der Lösenocken (32) ist,
wobei die Nockenachse (CA) des Lösenockens (32) in Bezug auf den Stator (2) feststehend ist und wobei der Lösenocken (32) dazu konfiguriert ist, sich um die Nockenachse bezogen auf den Stator (2) zu drehen,
wobei insbesondere die Nockenachse (CA) im Wesentlichen parallel zur Rotorachse (RA) ist.

4. Peristaltische Pumpe nach einem der vorangehenden Ansprüche, wobei das Löseelement (31), insbesondere der Lösenocken (32) und ganz besonders die Nockenachse (CA) des Lösenockens (32), in Bezug auf eine Radialrichtung, die senkrecht zur Rotorachse (RA) ist, zwischen der Rotorachse (RA) und dem Pumpenlaufring (3) angeordnet ist.

5. Peristaltische Pumpe nach einem der vorangehenden Ansprüche, wobei der Stator (2) eine Grundwand (2a) umfasst, wobei der Pumpenlaufring (3) in der Höhe von der Grundwand (2a) vorsteht und so ein Innenvolumen definiert, das in der Tiefe durch die Grundwand (2a) und seitlich durch den Pumpenlaufring (3) begrenzt wird, wobei der Rotor (10) wenigstens teilweise innerhalb des Innenvolumens des Stators (2) angeordnet ist,
wobei der Lösenocken (32) eine Lagerachse (32a), die um die Nockenachse (CA) drehbar ist, und ein nockenförmiges aufsitzendes Element (32b), das an einem Ende der Lagerachse (32a) befestigt ist, aufweist, wobei die Lagerachse (32a) in Kombination mit dem nockenförmigen aufsitzenden Element (32b) den Lösenocken (32) als einen einzigen Körper definiert,
und wobei die Grundwand (2a) des Stators (2) ein Durchgangsloch (4) umfasst, durch das hindurch die Lagerachse (32a) des Lösenockens (32) verlaufen kann, wobei das nockenförmige aufsitzende Element (32b) des Lösenockens (32) in der Höhe aus der Grundwand (2a) des Stators (2) heraus- und in das Innenvolumen hineinsteht und so eine nockenförmige seitliche Anlagewand (32c) definiert,
wobei:
- wenn der Lösenocken (32) sich in der aktiven Position befindet, die nockenförmige seitliche Anlagewand (32c) an dem wenigstens einen gegensätzlichen Element (11) des Rotors (10) anliegt, um die gelöste Konfiguration der peristaltischen Pumpe zu definieren, und
- wenn der Lösenocken (32) sich in der Ruheposition befindet, die nockenförmige seitliche Anlagewand (32c) erlaubt, dass das wenigstens eine gegensätzliche Element (11) in der Betriebsposition bleibt, um die Betriebskonfiguration der peristaltischen Pumpe zu definieren.

6. Peristaltische Pumpe nach einem der vorangehenden Ansprüche, wobei der Rotor (10) eine Rotorplatte (10a) umfasst, die das wenigstens eine gegensätzliche Element (11) trägt, und wobei jedes gegensätzliche Element (11) Folgendes aufweist:
- wenigstens eine(n) Rolle oder Gleitschuh (12), die/der dazu konfiguriert ist, im Betriebszustand der peristaltischen Pumpe mit dem Schlauch (50) in Kontakt zu stehen und diesen gegen den Pumpenlaufring (3) zusammenzudrücken;
- einen Hebelarm (13), der sich zwischen einem angelenkten Ende (13a) und einem freien Ende (13b) erstreckt, wobei der Hebelarm (13) um das angelenkte Ende (13a) um eine Gelenkachse (HA) drehbar ist, wobei:
o das angelenkte Ende (13a) an die Rotorplatte (10a) angelenkt ist, wodurch eine Drehung des Hebelarms (13) um die Gelenkachse (HA) erlaubt wird, und
o das freie Ende (13b) die/den wenigstens eine(n) Rolle oder Gleitschuh (12) trägt,
wobei eine Drehung des Hebelarms (13) um die Gelenkachse (HA) den Wechsel des jeweiligen gegensätzlichen Elements (11) zwischen der Betriebsposition und der gelösten Position bestimmt, wobei insbesondere der Abstand zwischen dem gegensätzlichen Element und dem Pumpenlaufring (3) als ein Abstand definiert ist, der zwischen der Rolle oder dem Gleitschuh (12) und dem Pumpenlaufring (3) vorliegt.

7. Peristaltische Pumpe nach dem vorangehenden Anspruch, wobei der Hebelarm (13) einen lang gestreckten Körper und einen Anlagevorsprung (14), der aus dem lang gestreckten Körper heraussteht, umfasst, wobei der Anlagevorsprung (14) dazu konfiguriert ist, an der nockenförmigen seitlichen Anlagewand (32c) des Lösenockens (32) anzuliegen, wenn der Lösenocken sich in der aktiven Position befindet, wobei der Anlagevorsprung (14) zwischen dem lang gestreckten Körper und der Grundwand (2a) des Stators (2) angeordnet ist.

8. Peristaltische Pumpe nach einem der vorangehenden Ansprüche, wobei die Pumpe (1) Folgendes umfasst:
- zwei oder mehr gegensätzliche Elemente (11) die einander in Bezug auf die Rotorachse (RA) im Wesentlichen diametral gegenüberliegen, wobei das Löseelement (31) dazu konfiguriert ist, jeweils ein gegensätzliches Element aus den zwei oder mehr gegensätzlichen Elementen (11) in Eingriff zu nehmen, oder
- drei oder mehr gegensätzliche Elemente (11), die in einer im Wesentlichen gleichmäßigen Weise voneinander winkelbeabstandet sind, wobei insbesondere die drei oder mehr gegensätzlichen Elemente gleich voneinander beabstandet sind, wobei das Löseelement (31) dazu konfiguriert ist, jeweils ein oder zwei gegensätzliche Elemente aus den drei oder mehr gegensätzlichen Elementen (11) in Eingriff zu nehmen.

9. Peristaltische Pumpe nach einem der vorangehenden Ansprüche, wobei das Schubelement (20) Folgendes umfasst:
- wenigstens ein elastisches Element (21), insbesondere eine Feder oder ein gummiartiges Element, ganz besonders eine Druck- oder Zugfeder, das auf das wenigstens eine gegensätzliche Element (11), insbesondere auf jedes gegensätzliche Element, einwirkt und dazu konfiguriert ist, das wenigstens eine gegensätzliche Element (11) in die Auswärtsrichtung zu bewegen, wobei das wenigstens eine elastische Element eine Bewegung des wenigstens einen gegensätzlichen Elements (11) aus der gelösten Position in die Betriebsposition bewirkt,
und wobei das Löseelement (31), insbesondere der Lösenocken (32), dazu konfiguriert ist, während eines Wechsels aus der Betriebskonfiguration in die gelöste Konfiguration eine Kraft auf das wenigstens eine gegensätzliche Element (11) auszuüben, die in ihrer Richtung einer Kraft, die durch das Schubelement (20) auf das wenigstens eine gegensätzliche Element (11) ausgeübt wird, entgegengesetzt ist;
oder
- einen Schubnocken (22), der durch Drehung bewegbar ist zwischen:
o einer Schubposition, in welcher der Schubnocken (22) an dem wenigstens einen gegensätzlichen Element (11) anliegt und so bewirkt, dass das gegensätzliche Element (11) sich in die Betriebsposition bewegt, und
o einer Nichtschubposition, in welcher der Schubnocken (22) erlaubt, dass das gegensätzliche Element (11) sich in die gelöste Position bewegt.

10. Peristaltische Pumpe nach dem vorangehenden Anspruch, wobei das elastische Element für Folgendes konfiguriert ist:
- Ausüben, wenn die Pumpe sich in der Betriebskonfiguration befindet, einer Kraft auf das wenigstens eine gegensätzliche Element (11), sodass das Letztgenannte in der Lage ist, den Schlauch (50) angemessen zusammenzudrücken, insbesondere zu verformen, um den Fluidfluss zu unterstützen, und
- sich selbst zu verformen, damit das wenigstens eine gegensätzliche Element (11) sich aus der Betriebsposition in die gelöste Position bewegen kann.

11. Peristaltische Pumpe nach einem der vorangehenden Ansprüche, wobei beim Umschalten zwischen der Betriebskonfiguration und der gelösten Konfiguration der Rotor (10) in einer einen vordefinierten Winkel aufweisenden Entriegelungsposition angeordnet wird,
wobei, wenn der Rotor (10) sich in der einen vordefinierten Winkel aufweisenden Entriegelungsposition befindet, wenigstens eines der gegensätzlichen Elemente (11) durch das Löseelement (31) in Eingriff nehmbar ist, um aus der Betriebsposition in die gelöste Position und umgekehrt umzuschalten,
wobei der Pumpenlaufring (3) Folgendes aufweist:
- eine Innenwand, die dazu konfiguriert ist, mit dem Schlauch in Kontakt zu stehen, wobei die Innenwand einen Betriebswinkelbogen für die gegensätzlichen Elemente definiert, wobei die gegensätzlichen Elemente der Innenwand des Pumpenlaufrings zugewandt sind, und
- eine seitliche Öffnung, die dazu konfiguriert ist zu erlauben, dass ein Einlassstrang des Schlauches (50) durch die Pumpe aufgenommen wird und ein Ausgangsstrang des Schlauches die Pumpe verlässt, wobei die seitliche Öffnung einen Nichtbetriebswinkelbogen für die gegensätzlichen Elemente definiert, wobei insbesondere die gegensätzlichen Elemente, wenn sie sich in dem Nichtbetriebswinkelbogen befinden, nicht dazu konfiguriert sind, vollständig mit dem Schlauch (50) zusammenzuarbeiten,
und wobei der Rotor (10), wenn er in der einen vordefinierten Winkel aufweisenden Entriegelungsposition angeordnet ist, Folgendes aufweist:
- wenigstens ein erstes gegensätzliches Element, das in dem Betriebswinkelbogen befindlich ist, wobei das wenigstens eine erste gegensätzliche Element durch das Löseelement (31) in Eingriff nehmbar ist; und
- wenigstens ein zweites gegensätzliches Element, das in dem Nichtbetriebswinkelbogen befindlich ist, wobei das wenigstens eine zweite gegensätzliche Element nicht durch das Löseelement (31) in Eingriff nehmbar ist,
wobei das Löseelement (31), wenn es in der einen vordefinierten Winkel aufweisenden Entriegelungsposition angeordnet ist, dazu konfiguriert ist, das wenigstens eine erste gegensätzliche Element in Eingriff zu nehmen, um das Letztgenannte in die gelöste Position zu bewegen, wobei insbesondere das Löseelement (31), wenn es aus der Ruheposition in die aktive Position wechselt, dazu konfiguriert ist, das wenigstens eine zweite gegensätzliche Element nicht in Eingriff zu nehmen.

12. Peristaltische Pumpe nach dem vorangehenden Anspruch, wobei die peristaltische Pumpe Folgendes umfasst:
- einen Antriebsmotor (60), der mit dem Rotor (10) wirkverbunden und dazu konfiguriert ist, den Rotor um die Rotorachse (RA) zu drehen, um einen Fluidfluss in dem Schlauch (50) zu bestimmen;
- einen Positionssensor (70), der dazu konfiguriert ist, ein Signal auszugeben, das eine Winkelposition des Rotors (10) in Bezug auf den Stator (2) angibt, wobei der Positionssensor insbesondere ein Hall-Sensor ist, der dazu konfiguriert ist, eine Position oder einen Vorbeilauf der Rolle oder des Gleitschuhs (12) zu erkennen;
- eine Steuereinheit (80), die mit dem Antriebsmotor (60) und dem Positionssensor (70) wirkverbunden ist, wobei die Steuereinheit dazu konfiguriert ist, das durch den Positionssensor (70) ausgegebene Signal zu empfangen und eine Winkelposition des Rotors in Bezug auf den Stator (2) oder eine Position wenigstens eines gegensätzlichen Elements (11) in Bezug auf das Löseelement (31) zu bestimmen,
und wobei die Steuereinheit dazu konfiguriert ist, Folgendes durchzuführen:
- ein Löseprozedere, das dazu konfiguriert ist, die peristaltische Pumpe aus der Betriebskonfiguration in die gelöste Konfiguration umzuschalten, wobei das Löseprozedere Folgendes umfasst:
o Anhalten oder Drehen des Rotors (10) in der/die einen vordefinierten Winkel aufweisende(n) Entriegelungsposition basierend auf der durch den Positionssensor (70) erkannten Position;
o Bewegen des Löseelements (31), insbesondere Drehen des Lösenockens (32), aus der Ruheposition in die aktive Position, um das wenigstens eine gegensätzliche Element (11) aus der Betriebsposition in die gelöste Position zu bewegen, wobei der Schritt des Bewegens des Löseelements (31) manuell durch eine Bedienperson oder durch die Steuereinheit (80) über einen Aktuator durchgeführt wird;
o Entfernen des Schlauches (50) zwischen dem wenigstens einen gegensätzlichen Element (11) und dem Pumpenlaufring (3);
und
- ein Einrichtungsprozedere im Anschluss an das Löseprozedere, das dazu konfiguriert ist, die peristaltische Pumpe aus der gelösten Konfiguration in die Betriebskonfiguration umzuschalten, wobei das Einrichtungsprozedere Folgendes umfasst:
o Anhalten oder Drehen des Rotors (10) in der/die einen vordefinierten Winkel aufweisende(n) Entriegelungsposition basierend auf der durch den Positionssensor (70) erkannten Position;
o Bewegen des Löseelements (31), insbesondere Drehen des Lösenockens (32), aus der Ruheposition in die aktive Position, um das wenigstens eine gegensätzliche Element (11) aus der Betriebsposition in die gelöste Position zu bewegen, wobei der Schritt des Bewegens des Löseelements (31) manuell durch eine Bedienperson oder durch die Steuereinheit (80) über einen Aktuator durchgeführt wird;
o Installieren des Schlauches (50) zwischen dem wenigstens einen gegensätzlichen Element (11) und dem Pumpenlaufring (3);
o Bewegen des Löseelements (31), insbesondere Drehen des Lösenockens (32), aus der aktiven Position in die Ruheposition, um das wenigstens eine gegensätzliche Element (11) aus der gelösten Position in die Betriebsposition zu bewegen, wobei der Schritt des Bewegens des Löseelements (31) manuell durch eine Bedienperson oder durch die Steuereinheit (80) über einen Aktuator durchgeführt wird,
wobei insbesondere die Schritte des Installierens und Entfernens des Schlauches (50) in die/aus der peristaltische(n) Pumpe (1) durch einen Benutzer durchgeführt werden.

13. Peristaltische Pumpe nach einem der vorangehenden Ansprüche, wobei das Lösesystem (30) einen Positionssensor (71) umfasst, der mit der Steuereinheit (80) wirkverbunden und dazu konfiguriert ist, ein Signal auszugeben, das die aktive Position oder die Ruheposition des Löseelements (31) angibt, wobei die Steuereinheit (80) dazu konfiguriert ist, selektiv zu bestimmen, ob das Löseelement (31) sich in der aktiven Position oder der Ruheposition befindet,
wobei die Pumpe ferner einen Aktuator umfasst, der mit der Steuereinheit (80) und mit dem Löseelement (31) wirkverbunden ist, wobei der Aktuator dazu konfiguriert ist, auf Befehl der Steuereinheit (80) das Löseelement (31) zwischen der Ruheposition und der aktiven Position zu bewegen,
und wobei die Steuereinheit (80) für Folgendes konfiguriert ist:
- Erkennen der Position des Löseelements (31) durch den Positionssensor (71) des Lösesystems (30);
- Einschätzen, ob die erkannte Position des Löseelements (31) der aktiven Position oder der Ruheposition entspricht;
- wenn erkannt wird, dass das Löseelement (31) sich in der aktiven Position befindet, Verhindern einer Drehung des Rotors (10) durch Unterbinden der Drehung des Antriebsmotors (60) des Rotors (10).

14. Peristaltische Pumpe nach einem der vorangehenden Ansprüche, wobei der Abstand zwischen dem wenigstens einen gegensätzlichen Element (11) und dem Pumpenlaufring (3) als ein Abstand definiert ist, der zwischen einem Abschnitt des gegensätzlichen Elements, das dazu konfiguriert ist, an dem Schlauch (50) anzuliegen, und dem Pumpenlaufring (3) vorliegt,
wobei insbesondere der Abstand zwischen dem wenigstens einen gegensätzlichen Element (11) und dem Pumpenlaufring (3) als ein Abstand definiert ist, der zwischen einer Rolle oder einem Gleitschuh (12), wenn dieser in dem Betriebsbogen des Pumpenlaufrings (3) angeordnet ist, und dem Pumpenlaufring (3) vorliegt,
und wobei das Löseelement (31) sich nicht mit dem Rotor (10) um die Rotorachse(RA) dreht und wobei während eines Arbeitszustands der peristaltischen Pumpe (1), insbesondere während einer Drehung des Rotors (10) um die Rotorachse (RA), das Löseelement (21) bezogen auf den Stator (2) feststehend ist und der Rotor (10) dazu konfiguriert ist, sich in Bezug auf das Löseelement (31) zu drehen.

15. Verfahren (1000) zum Umschalten der peristaltischen Pumpe (1) aus der Betriebskonfiguration in die gelöste Konfiguration und umgekehrt, wobei die peristaltische Pumpe (1) einem der vorangehenden Ansprüche entspricht, wobei das Verfahren zum Umschalten der peristaltischen Pumpe (1) aus der Betriebskonfiguration in die gelöste Konfiguration wenigstens folgende Schritte umfasst:
- Anhalten oder Drehen des Rotors (10) in eine(r) einen vordefinierten Winkel aufweisende(n) Entriegelungsposition in Bezug auf den Stator (2), wobei die einen vordefinierten Winkel aufweisende Entriegelungsposition als eine Position definiert ist, in der wenigstens eines der gegensätzlichen Elemente (11) durch das Löseelement (31) in Eingriff nehmbar ist, um aus der Betriebsposition in die gelöste Position und umgekehrt umzuschalten;
- Bewegen des Löseelements (31), insbesondere Drehen eines Lösenockens (32), aus der Ruheposition in die aktive Position, um das wenigstens eine gegensätzliche Element (11) aus der Betriebsposition in die gelöste Position zu bewegen, wobei der Schritt des Bewegens des Löseelements (31) manuell durch eine Bedienperson oder durch eine Steuereinheit (80) über einen Aktuator durchgeführt wird;
und wobei das Verfahren zum Umschalten der peristaltischen Pumpe (1) aus der gelösten Konfiguration in die Betriebskonfiguration wenigstens folgende Schritte umfasst:
- Anhalten oder Drehen des Rotors (10) in eine(r) einen vordefinierten Winkel aufweisende(n) Entriegelungsposition in Bezug auf den Stator (2), wobei die einen vordefinierten Winkel aufweisende Entriegelungsposition als eine Position definiert ist, in der wenigstens eines der gegensätzlichen Elemente (11) durch das Löseelement (31) in Eingriff nehmbar ist, um aus der Betriebsposition in die gelöste Position und umgekehrt umzuschalten;
- Bewegen des Löseelements (31), insbesondere Drehen des Lösenockens (32), aus der aktiven Position in die Ruheposition, um das wenigstens eine gegensätzliche Element (11) aus der gelösten Position in die Betriebsposition zu bewegen, wobei der Schritt des Bewegens des Löseelements (31) manuell durch eine Bedienperson oder durch die Steuereinheit (80) über einen Aktuator durchgeführt wird.

## Revendications

1. Pompe péristaltique (1) configurée pour recevoir une ligne de fluide (50), ladite pompe péristaltique comprenant :
- un stator (2) comprenant un chenal de pompe (3) ;
- un rotor (10) entouré au moins partiellement par le chenal de pompe (3) et configuré pour tourner autour d'un axe de rotor (RA), ledit rotor (10) comprenant :
o au moins un élément **d'opposition** (11) configuré pour venir en butée contre la ligne de fluide (50), l'au moins un élément d'opposition (11) étant mobile en rapprochement et en éloignement par rapport à l'axe de rotor (RA) et au chenal de pompe (3) entre :
▪ une position libérée, l'au moins un élément d'opposition (11) étant éloigné du chenal de pompe (3), et
▪ une position fonctionnelle, l'au moins un élément d'opposition (11) étant rapproché du chenal de pompe (3) et configuré pour venir en butée contre la ligne de fluide (50), dans la position libérée l'au moins un élément d'opposition (11) étant à une distance du chenal de pompe (3) supérieure à celle de la position fonctionnelle,
o un élément de poussée (20) configuré pour agir, au moins dans la position fonctionnelle, en poussée sur ledit élément d'opposition (11) dans une direction vers l'extérieur en direction du chenal de pompe (3) ; et
- un système de libération (30) comprenant un élément de libération (31) mobile entre une position active et une position de repos ;
la pompe péristaltique (1) étant configurable dans au moins :
- une configuration fonctionnelle, l'élément de libération (31) étant déplacé dans la position de repos pour permettre à l'au moins un élément d'opposition (11) de se déplacer dans la position fonctionnelle, l'élément d'opposition (11) étant poussé par l'élément de poussée (20) dans la direction vers l'extérieur ; et
- une configuration libérée, l'élément de libération (31) étant déplacé dans la position active, ledit élément de libération (31) venant en prise avec l'élément d'opposition (11) et déplaçant l'élément d'opposition (11) dans la position libérée à distance du chenal de pompe (3) ;
**caractérisé en ce que** l'élément de libération (31) est porté par le stator (2) et est décalé spatialement par rapport à l'axe de rotor (RA).

2. Pompe péristaltique selon la revendication précédente,
- l'élément de libération (31) étant une came de libération (32) rotative autour d'un axe de came (CA), ledit axe de came étant décalé spatialement par rapport à l'axe de rotor (RA), et :
o dans la configuration fonctionnelle, la came de libération (32) étant tournée dans la position de repos pour permettre à l'au moins un élément d'opposition (11) de rester dans la position fonctionnelle, l'élément d'opposition (11) étant poussé par l'élément de poussée (20) dans la direction vers l'extérieur ;
o dans la configuration libérée, la came de libération (32) étant tournée dans la position active, ladite came de libération (32) venant en prise avec l'au moins un élément d'opposition (11) et maintenant l'au moins un élément d'opposition (11) dans la position libérée à distance du chenal de pompe (3) ;
ou
- l'élément de libération (31) étant un élément de translation mobile par translation le long d'une trajectoire rectiligne ou curviligne,
o dans la configuration fonctionnelle, l'élément de translation étant déplacé dans la position de repos pour permettre à l'au moins un élément d'opposition (11) de rester dans la position fonctionnelle, l'élément d'opposition (11) étant poussé par l'élément de poussée (20) dans la direction vers l'extérieur ;
o dans la configuration libérée, l'élément de translation étant déplacé dans la position active, ledit élément de translation venant en prise avec l'au moins un élément d'opposition (11) et maintenant l'au moins un élément d'opposition (11) dans la position libérée à distance du chenal de pompe (3).

3. Pompe péristaltique selon la revendication précédente, l'élément de libération (31) étant la came de libération (32),
l'axe de came (CA) de la came de libération (32) étant fixe par rapport au stator (2), et la came de libération (32) étant configurée pour tourner autour de l'axe de came par rapport au stator (2),
en particulier l'axe de came (CA) étant sensiblement parallèle à l'axe de rotor (RA).

4. Pompe péristaltique selon l'une quelconque des revendications précédentes, l'élément de libération (31), en particulier la came de libération (32) et plus particulièrement l'axe de came (CA) de la came de libération (32), étant interposé entre l'axe de rotor (RA) et le chenal de pompe (3) par rapport à une direction radiale normale à l'axe de rotor (RA).

5. Pompe péristaltique selon l'une quelconque des revendications précédentes, le stator (2) comprenant une paroi de base (2a), le chenal de pompe (3) faisant saillie en hauteur depuis ladite paroi de base (2a) définissant un volume interne confiné en profondeur par la paroi de base (2a) et latéralement par le chenal de pompe (3), le rotor (10) étant agencé au moins partiellement dans ledit volume interne du stator (2),
la came de libération (32) comprenant un axe de support (32a) rotatif autour de l'axe de came (CA), et un élément supérieur en forme de came (32b) fixé à une extrémité de l'axe de support (32a), l'axe de support (32a) en combinaison avec l'élément supérieur en forme de came (32b) définissant la came de libération (32) comme un corps unique,
et la paroi de base (2a) du stator (2) comprenant un trou traversant (4) permettant le passage de l'axe de support (32a) de la came de libération (32), l'élément supérieur en forme de came (32b) de la came de libération (32) émergeant en hauteur depuis ladite paroi de base (2a) du stator (2) dans le volume interne définissant une paroi de butée latérale en forme de came (32c),
dans laquelle :
- lorsque la came de libération (32) est dans la position active, ladite paroi de butée latérale en forme de came (32c) venant en butée contre l'au moins un élément d'opposition (11) du rotor (10) pour définir la configuration libérée de la pompe péristaltique, et
- lorsque la came de libération (32) est dans la position de repos, ladite paroi de butée latérale en forme de came (32c) permettant à l'au moins un élément d'opposition (11) de rester dans la position fonctionnelle pour définir la configuration fonctionnelle de la pompe péristaltique.

6. Pompe péristaltique selon l'une quelconque des revendications précédentes, le rotor (10) comprenant une plaque de rotor (10a) portant l'au moins un élément d'opposition (11), et chaque élément d'opposition (11) comprenant :
- au moins un galet ou patin (12) configuré pour venir en contact avec et comprimer la ligne de fluide (50) contre le chenal de pompe (3) dans la condition fonctionnelle de la pompe péristaltique ;
- un bras de levier (13) s'étendant entre une extrémité articulée (13a) et une extrémité libre (13b), ledit bras de levier (13) étant rotatif autour de l'extrémité articulée (13a) autour d'un axe d'articulation (HA),
o l'extrémité articulée (13a) étant articulée à ladite plaque de rotor (10a) permettant ainsi la rotation du bras de levier (13) autour de l'axe d'articulation (HA), et
o l'extrémité libre (13b) portant l'au moins un galet ou patin (12),
une rotation du bras de levier (13) autour de l'axe d'articulation (HA) déterminant le passage de l'élément d'opposition (11) respectif entre la position fonctionnelle et la position libérée, en particulier la distance entre l'élément d'opposition et le chenal de pompe (3) étant définie comme une distance interposée entre le galet ou patin (12) et le chenal de pompe (3).

7. Pompe péristaltique selon la revendication précédente, le bras de levier (13) comprenant un corps étendu et une saillie de butée (14) émergeant dudit corps étendu, ladite saillie de butée (14) étant configurée pour venir en butée contre la paroi de butée latérale en forme de came (32c) de la came de libération (32) lorsque la came de libération est dans la position active, ladite saillie de butée (14) étant interposée entre ledit corps étendu et la paroi de base (2a) du stator (2).

8. Pompe péristaltique selon l'une quelconque des revendications précédentes, la pompe (1) comprenant :
- deux ou plus de deux éléments d'opposition (11) sensiblement diamétralement opposés l'un par rapport à l'autre par rapport à l'axe de rotor (RA), l'élément de libération (31) étant configuré pour venir en prise avec un élément d'opposition à la fois parmi les deux ou plus de deux éléments d'opposition (11), ou
- trois ou plus de trois éléments d'opposition (11) espacés angulairement les uns des autres de manière sensiblement régulière, en particulier les trois ou plus de trois éléments d'opposition étant espacés de façon égale les uns des autres, l'élément de libération (31) étant configuré pour venir en prise avec un ou deux éléments d'opposition à la fois parmi les trois ou plus de trois éléments d'opposition (11).

9. Pompe péristaltique selon l'une quelconque des revendications précédentes, l'élément de poussée (20) comprenant :
- au moins un élément élastique (21), en particulier un ressort ou un élément de type caoutchouc, plus particulièrement un ressort de compression ou de traction, agissant sur l'au moins un élément d'opposition (11), en particulier sur chaque élément d'opposition, et configuré pour déplacer l'au moins un élément d'opposition (11) dans la direction vers l'extérieur, ledit au moins un élément élastique provoquant le déplacement de l'au moins un élément d'opposition (11) de la position libérée vers la position fonctionnelle,
et l'élément de libération (31), en particulier la came de libération (32), étant configuré, lors d'un passage entre la configuration fonctionnelle et la configuration libérée, pour exercer une force sur l'au moins un élément d'opposition (11) de direction opposée à une force exercée par l'élément de poussée (20) sur l'au moins un élément d'opposition (11) ;
ou
- une came de poussée (22) mobile par rotation entre :
o une position de poussée, la came de poussée (22) venant en butée contre l'au moins un élément d'opposition (11) provoquant le déplacement de l'élément d'opposition (11) dans la position fonctionnelle, et
o une position de non-poussée, la came de poussée (22) permettant à l'élément d'opposition (11) de se déplacer dans la position libérée.

10. Pompe péristaltique selon la revendication précédente, l'élément élastique étant configuré pour :
- exercer, lorsque la pompe est dans la configuration fonctionnelle, une force sur l'au moins un élément d'opposition (11) de telle sorte que ce dernier puisse comprimer de manière adéquate, en particulier déformer, la ligne de fluide (50) pour favoriser l'écoulement de fluide, et
- se déformer pour permettre à l'au moins un élément d'opposition (11) de se déplacer de la position fonctionnelle vers la position libérée.

11. Pompe péristaltique selon l'une quelconque des revendications précédentes, lors du passage entre la configuration fonctionnelle et la configuration libérée, le rotor (10) étant agencé dans une position angulaire de déverrouillage prédéfinie,
lorsque le rotor (10) est dans ladite position angulaire de déverrouillage prédéfinie, au moins l'un des éléments d'opposition (11) pouvant venir en prise avec l'élément de libération (31) pour passer de la position fonctionnelle à la position libérée et vice versa,
le chenal de pompe (3) comportant :
- une paroi interne configurée pour venir en contact avec la ligne de fluide, ladite paroi interne définissant un arc angulaire fonctionnel pour les éléments d'opposition, lesdits éléments d'opposition étant orientés vers ladite paroi interne du chenal de pompe, et
- une ouverture latérale configurée pour permettre à un tronçon d'entrée de la ligne de fluide (50) d'être reçu par la pompe, et à un tronçon de sortie de la ligne de fluide de sortir de la pompe, ladite ouverture latérale définissant un arc angulaire non fonctionnel pour les éléments d'opposition, en particulier les éléments d'opposition, lorsqu'ils sont dans ledit arc angulaire non fonctionnel, n'étant pas configurés pour coopérer pleinement avec la ligne de fluide (50),
et le rotor (10), lorsqu'il est agencé dans la position angulaire de déverrouillage prédéfinie, comportant :
- au moins un premier élément d'opposition positionné dans l'arc angulaire fonctionnel, ledit au moins un premier élément d'opposition pouvant venir en prise avec l'élément de libération (31) ; et
- au moins un second élément d'opposition positionné dans ledit arc angulaire non fonctionnel, ledit au moins un second élément d'opposition ne pouvant pas venir en prise avec l'élément de libération (31),
l'élément de libération 31, lorsqu'il est agencé dans la position angulaire de déverrouillage prédéfinie, étant configuré pour venir en prise avec ledit au moins un premier élément d'opposition pour déplacer ce dernier dans la position libérée,
en particulier l'élément de libération 31, lors du passage de la position de repos à la position active, étant configuré pour ne pas venir en prise avec ledit au moins un second élément d'opposition.

12. Pompe péristaltique selon la revendication précédente, la pompe péristaltique comprenant :
- un moteur d'entraînement (60) relié fonctionnellement au rotor (10) et configuré pour faire tourner ledit rotor autour de l'axe de rotor (RA) pour déterminer un écoulement de fluide dans la ligne de fluide (50) ;
- un capteur de position (70) configuré pour émettre un signal représentatif d'une position angulaire du rotor (10) par rapport au stator (2), ledit capteur de position étant en particulier un capteur à effet Hall configuré pour détecter une position ou un passage du galet ou patin (12) ;
- une unité de commande (80) reliée fonctionnellement au moteur d'entraînement (60) et au capteur de position (70), l'unité de commande étant configurée pour recevoir le signal émis par le capteur de position (70) et pour déterminer une position angulaire du rotor par rapport au stator (2) ou une position d'au moins un élément d'opposition (11) par rapport à l'élément de libération (31),
et l'unité de commande étant configurée pour réaliser :
- une procédure de libération, configurée pour faire passer la pompe péristaltique de la configuration fonctionnelle à la configuration libérée, ladite procédure de libération comprenant :
∘ l'arrêt ou la rotation du rotor (10) jusqu'à la position angulaire de déverrouillage prédéfinie sur la base de la position détectée par le capteur de position (70) ;
o le déplacement de l'élément de libération (31), en particulier la rotation de la came de libération (32), de la position de repos à la position active pour déplacer l'au moins un élément d'opposition (11) de la position fonctionnelle à la position libérée, ladite étape de déplacement de l'élément de libération (31) étant réalisé manuellement par un opérateur ou par l'unité de commande (80) au moyen d'un actionneur ;
o le retrait de la ligne de fluide (50) entre l'au moins un élément d'opposition (11) et le chenal de pompe (3) ;
et
- une procédure d'installation, consécutive à la procédure de libération, configurée pour faire passer la pompe péristaltique de la configuration libérée à la configuration fonctionnelle, ladite procédure d'installation comprenant :
o l'arrêt ou la rotation du rotor (10) jusqu'à la position angulaire de déverrouillage prédéfinie sur la base de la position détectée par le capteur de position (70) ;
o le déplacement de l'élément de libération (31), en particulier la rotation de la came de libération (32), de la position de repos à la position active pour déplacer l'au moins un élément d'opposition (11) de la position fonctionnelle à la position libérée, ladite étape de déplacement de l'élément de libération (31) étant réalisée manuellement par un opérateur ou par l'unité de commande (80) au moyen d'un actionneur ;
o l'installation de la ligne de fluide (50) entre l'au moins un élément d'opposition (11) et le chenal de pompe (3)
o le déplacement de l'élément de libération (31), en particulier la rotation de la came de libération (32), de la position active à la position de repos pour déplacer l'au moins un élément d'opposition (11) de la position libérée à la position fonctionnelle, ladite étape de déplacement de l'élément de libération (31) étant réalisée manuellement par un opérateur ou par l'unité de commande (80) au moyen d'un actionneur,
en particulier, les étapes d'installation et de retrait de la ligne de fluide (50) de la pompe péristaltique (1) étant réalisées par un utilisateur.

13. Pompe péristaltique selon l'une quelconque des revendications précédentes, le système de libération (30) comprenant un capteur de position (71) relié fonctionnellement à l'unité de commande (80) et configuré pour émettre un signal représentatif de la position active ou de repos de l'élément de libération (31), ladite unité de commande (80) étant configurée pour déterminer sélectivement si l'élément de libération (31) est dans la position active ou de repos, la pompe comprenant en outre un actionneur relié fonctionnellement à l'unité de commande (80) et à l'élément de libération (31), l'actionneur étant configuré pour déplacer l'élément de libération (31) entre la position de repos et la position active sur commande de l'unité de commande (80),
et l'unité de commande (80) étant configurée pour :
- détecter la position de l'élément de libération (31) par le capteur de position (71) du système de libération (30) ;
- évaluer si ladite position détectée de l'élément de libération (31) correspond à la position active ou à la position de repos ;
- si l'élément de libération (31) est détecté comme étant dans la position active, empêcher la rotation du rotor (10) en inhibant la rotation du moteur d'entraînement (60) du rotor (10).

14. Pompe péristaltique selon l'une quelconque des revendications précédentes, la distance entre l'au moins un élément d'opposition (11) et le chenal de pompe (3) étant définie comme une distance interposée entre une partie de l'élément d'opposition configurée pour venir en butée contre la ligne de fluide (50) et le chenal de pompe (3),
en particulier, la distance entre l'au moins un élément d'opposition (11) et le chenal de pompe (3) étant définie comme une distance interposée entre un galet ou patin (12), lorsqu'il est agencé dans l'arc fonctionnel du chenal de pompe (3), et le chenal de pompe (3),
et l'élément de libération (31) ne tournant pas avec le rotor (10) autour de l'axe de rotor (RA), et, pendant une condition de fonctionnement de la pompe péristaltique (1), en particulier pendant une rotation du rotor (10) autour de l'axe de rotor (RA), l'élément de libération (21) étant fixe par rapport au stator (2) et le rotor (10) étant configuré pour tourner par rapport à l'élément de libération (31).

15. Procédé (1000) pour faire passer la pompe péristaltique (1) de la configuration fonctionnelle à la configuration libérée et vice versa, ladite pompe péristaltique (1) étant selon l'une quelconque des revendications précédentes,
le procédé pour faire passer la pompe péristaltique (1) de la configuration fonctionnelle à la configuration libérée comprenant au moins les étapes suivantes :
- l'arrêt ou la rotation du rotor (10) jusqu'à une position angulaire de déverrouillage prédéfinie par rapport au stator (2), la position angulaire de déverrouillage prédéfinie étant définie comme une position dans laquelle au moins l'un des éléments d'opposition (11) peut venir en prise avec l'élément de libération (31) pour passer de la position fonctionnelle à la position libérée et vice versa ;
- le déplacement de l'élément de libération (31), en particulier la rotation d'une came de libération (32), de la position de repos à la position active pour déplacer l'au moins un élément d'opposition (11) de la position fonctionnelle à la position libérée, ladite étape de déplacement de l'élément de libération (31) étant réalisée manuellement par un opérateur ou par une unité de commande (80) au moyen d'un actionneur ;
et le procédé pour faire passer la pompe péristaltique (1) de la configuration libérée à la configuration fonctionnelle comprenant au moins les étapes suivantes :
- l'arrêt ou la rotation du rotor (10) jusqu'à une position angulaire de déverrouillage prédéfinie par rapport au stator (2), la position angulaire de déverrouillage prédéfinie étant définie comme une position dans laquelle au moins l'un des éléments d'opposition (11) peut venir en prise avec l'élément de libération (31) pour passer de la position fonctionnelle à la position libérée et vice versa ;
- le déplacement de l'élément de libération (31), en particulier la rotation de la came de libération (32), de la position active à la position de repos pour déplacer l'au moins un élément d'opposition (11) de la position libérée à la position fonctionnelle, ladite étape de déplacement de l'élément de libération (31) étant réalisée manuellement par un opérateur ou par l'unité de commande (80) au moyen d'un actionneur.
